# EUROPEAN PATENT APPLICATION

(11) **EP 4 071 228 A1**
(43) Date of publication of application: **12.10.2022**
(21) Application number: 20897490.7
(22) Date of filing: 26.11.2020
(51) Int. Cl.: C12M 1/00, C12M 1/34, G01N 33/483, G01N 33/50, G01N 27/00

(54) **NANOPORE STRUCTURE AND BASE SEQUENCE ANALYSIS DEVICE INCLUDING NANOPORE STRUCTURE**

(30) Priority: 03.12.2019 JP 2019219169
(71) Applicant: Japan Science and Technology Agency, Kawaguchi-shi, Saitama 332-0012 (JP)
(72) Inventor: MAJIMA Yutaka, Yokohama-shi, Kanagawa 226-8503 (JP); PHAN Trong Tue, Yokohama-shi, Kanagawa 226-8503 (JP)
(74) Representative: dompatent von Kreisler Selting Werner - Partnerschaft von Patent- und Rechtsanwälten mbB
(86) International application number: PCT/JP2020/044075
(87) International publication number: WO 2021/111987

(57) **Abstract**

This nanopore structure: includes a first metal member in the form of a thin film that comprises through-holes and a second metal member that is disposed so as to narrow the diameters of the through-holes; and includes nanopores with diameters of 10 nm or less due to the first metal member and the second metal member. Moreover, this base sequence analysis device comprises a cis-chamber and a trans-chamber, a nanopore structure between the cis-chamber and the trans-chamber, a first electrode (working electrode) disposed in the cis-chamber, a second electrode (counter electrode) disposed in the trans-chamber, and a third electrode (reference electrode 2).

## Description

### TECHNICAL FIELD

An embodiment of the present invention relates to a nanopore structure having a nanoscale through hole formed of a thin metal film, and a device for analyzing a base sequence in DNA, RNA or the like including the nanopore structure.

### BACKGROUND ART

Development of devices and methods for DNA and RNA sequencing using nanoscale pores, called nanopores, is underway. For example, a base sequence analyzer (DNA sequencer) that forms nanopores with ring-shaped proteins and measures ion currents when DNA strands pass through the nanopores to decode base sequences is disclosed (refer to Patent Literature 1).

### CITATION LIST

### PATENT LITERATURE

Patent Literature 1: JP 2015-535179 A

### SUMMARY OF INVENTION

### TECHNICAL TO PROBLEM

The nanopore disclosed in PTL1 has problems in mechanical durability, stability, and heat resistance due to the use of protein. There is also a problem that nanopores formed by proteins cannot be reused once they are used for analysis. On the other hand, although attempts have been made to form nanopores using metal materials or semiconductor materials, it is difficult to manufacture nanopores having a pore diameter of 10 nm or less even by the most advanced microfabrication technology, and practical use has not yet been achieved.

In view of these problems, it is one of the object of the present invention to provide nanopore structures having a new structure, which are fabricated by an approach different from conventional techniques. It is also one of the object of the present invention to provide a base sequence analyzer using the nanopore structure having a new structure.

### SOLUTION TO PROBLEM

The present invention achieves nanopores having a pore diameter of 10 nm or less and a base sequence analyzer using the nanopores by applying an electrochemical method in addition to microfabrication techniques such as photolithography and etching.

A nanopore structure in an embodiment according to the present invention includes a first metal member having a thin film structure and a through hole, and a second metal member arranged to narrow a diameter of the through hole. The first metal member and the second metal member form a nanopore having a pore diameter of 10 nm or less.

A base sequence analyzer in an embodiment according to the present invention includes a cis chamber and a trans chamber, a nanopore structure partitioning the cis chamber and the trans chamber, a first electrode (working electrode) arranged in the cis chamber, and a second electrode (counter electrode) and a third electrode (reference electrode 1) arranged in the trans chamber. The nanopore structure includes a first metal member having a thin film structure and a through hole, and a second metal member arranged to narrow a diameter of the through hole. The first metal member and the second metal member form a nanopore having a pore diameter of 10 nm or less.

### ADVANTAGEOUS EFFECTS OF INVENTION

According to an embodiment of the present invention, the nanopore structure formed of inorganic materials can be provided by combining plural kinds of metallic materials. More specifically, it is possible to provide the nanopore structure having nanopores with a pore diameter of 10 nm or less by forming the second metal member at the open end of the through hole of the first metal member by an electroless plating reaction. Further, it is possible to provide the base sequence analyzer using the same.

### BRIEF DESCRIPTION OF DRAWINGS

FIG. 1A shows a plan view of a nanopore structure according to an embodiment of the present invention.
FIG. 1B shows a cross-sectional view of a nanopore structure according to an embodiment of the present invention.
FIG. 2A shows a cross-sectional structure of a nanopore structure according to an embodiment of the present invention.
FIG. 2B shows a cross-sectional structure of a nanopore structure according to an embodiment of the present invention.
FIG. 3A shows a plan view of a nanopore structure according to an embodiment of the present invention.
FIG. 3B shows a cross-sectional view of a nanopore structure according to an embodiment of the present invention.
FIG. 4 shows a cross-sectional structure of a nanopore structure according to an embodiment of the present invention.
FIG. 5A shows a cross-sectional structure of a nanopore structure according to an embodiment of the present invention, and shows a configuration in which a self-assembled monolayer is formed on a first metal member and a second metal member.
FIG. 5B shows a cross-sectional structure of a nanopore structure according to an embodiment of the present invention, and shows a configuration in which, a self-assembled monolayer is formed in a nanopore of a second metal member.
FIG. 6 shows a plan view of a nanopore structure according to an embodiment of the present invention.
FIG. 7 shows a cross-sectional structure of a nanopore structure according to an embodiment of the present invention.
FIG. 8A shows a method of manufacturing a nanopore structure according to an embodiment of the present invention.
FIG. 8B shows a method of manufacturing a nanopore structure according to an embodiment of the present invention.
FIG. 8C shows a method of manufacturing a nanopore structure according to an embodiment of the present invention.
FIG. 9A shows a method of manufacturing a nanopore structure according to an embodiment of the present invention.
FIG. 9B shows a method of manufacturing a nanopore structure according to an embodiment of the present invention.
FIG. 9C shows a method of manufacturing a nanopore structure according to an embodiment of the present invention.
FIG. 10A shows a method of manufacturing a nanopore structure according to an embodiment of the present invention.
FIG. 10B shows a method of manufacturing a nanopore structure according to an embodiment of the present invention.
FIG. 10C shows a method of manufacturing a nanopore structure according to an embodiment of the present invention.
FIG. 11 shows a configuration of a base sequence analyzer according to an embodiment of the present invention.
FIG. 12A is an illustration for explaining a measurement mode of a base sequence analyzer according to an embodiment of the present invention, and schematically shows a measurement principle of an ion current.
FIG. 12B is an illustration for explaining a measurement mode of a base sequence analyzer according to an embodiment of the present invention, and schematically shows a measurement principle of a tunnel current.
FIG. 13 shows a configuration of a base sequence analyzer according to an embodiment of the present invention.
FIG. 14 shows a configuration of a base sequence analyzer according to an embodiment of the present invention.
FIG. 15 shows an example of a selection circuit for switching a plurality of electrodes with nanopores and connection points of the electrodes with nanopores in a base sequence analyzer according to an embodiment of the present invention.
FIG. 16 shows a timing chart for explaining the operation of the base sequence analyzer according to an embodiment of the present invention.
FIG. 17 shows SEM images of a sample prepared in Example 1.
FIG. 18 shows SEM images of a sample prepared in Example 2.

### DESCRIPTION OF EMBODIMENTS

Hereinafter, embodiments of the present invention will be described with reference to the drawings and the like. The present invention may be carried out in various forms without departing from the gist thereof, and is not to be construed as being limited to any of the following embodiments. Although the drawings may schematically represent the width, thickness, shape, and the like of each part in comparison with the actual embodiment in order to clarify the description, they are merely examples and do not limit the interpretation of the present invention. In the present specification and each of the figures, elements similar to those described previously with respect to the figures already mentioned are designated by the same reference numerals (or numbers followed by a, b, etc.), and a detailed description thereof may be omitted as appropriate. Furthermore, the characters "first" and "second" appended to each element are convenient signs used to distinguish each element, and have no further meaning unless specifically described.

### 1. Nanopore Structure

A nanopore structure according to an embodiment of the present invention is formed of a first metal member having a through hole and a second metal member formed to narrow the through hole. The details of the nanopore structure will be described below.

### 1-1. First Embodiment

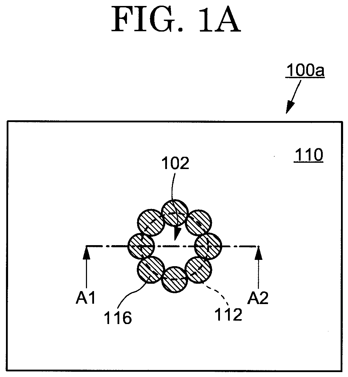

FIG. 1 A shows a schematic plan view of a nanopore structure 100a according to an embodiment of the present invention. The nanopore structure 100a includes a first metal member 110 and a second metal member 116, and has a structure in which a through hole 112 is formed in the first metal member 110. The nanopore structure 100a has a second metal member 116 formed on the first metal member 110 to narrow the through hole 112, thereby forming a nanopore 102.

The first metal member 110 is a thin plate and has a predetermined thickness. The through hole 112 is a hole in which the first metal member 110 is removed, and a side wall of the through hole 112 exposes the first metal member 110. The shape of the through hole 112 is arbitrary, and it can be formed in various shapes such as a circle, an ellipse, a triangle, a square or a rectangle in a plan view. The size of hole diameter of the through hole 112 is 8 nm to 40 nm, and this size indicates the diameter if the through hole 112 is circular and indicates diagonal length if it is square. When viewed as a single unit, the through hole 112 has a size relatively larger than a size suitable for functioning as a nanopore for analyzing a base sequence of DNA or the like.

The second metal member 116 may have a variety of shapes, such as an isolated island-like structure, an aggregated and continuous island-like structure, or a membrane-like continuous structure. FIG. 1A shows an embodiment in which the second metal member 116 is formed in an island-like. The second metal member 116 is formed so that at least one of the second metal members 116 overlaps the open end of the through hole 112. As shown in FIG. 1 A, a plurality of the second metal members 116 may be connected to each other to surround the end portion of the through hole 112. The second metal member 116 is arranged to protrude into the hole portion of the through hole 112. The through hole 112 is narrowed in diameter by the second metal member 116. The nanopore 102 is formed in a portion where the through hole 112 is narrowed by the second metal member 116. That is, the nanopore 102 is formed by combining the through hole 112 formed in the first metal member 110 and the second metal member 116. Although the second metal member 116 may be formed at any part of the surface of the first metal member 110 other than the through hole 112, it is not shown because it is not related to the nanopore 102.

As described above, the nanopore structure 100a has a nanopore 102 formed by the first metal member 110 having the through hole 112 and the second metal member 116 formed to narrow the through hole 112. The pore diameter of the nanopore in the present invention refers to the diameter of the pore formed by the second metal member. In the case where the nanopore is circular in a plan view, the diameter thereof shall be indicated, and in the case where the nanopore has a shape other than a circle or circular shape, the diameter obtained by converting the size of the opening obtained by {2 × (area in a plan view of the opening formed by the second metal member/pi)} 1/2 into a circle shall be referred to as the pore diameter of the nanopore. The nanopore 102 has a substantial pore size of 10 nm or less, preferably 1 nm to 5 nm.

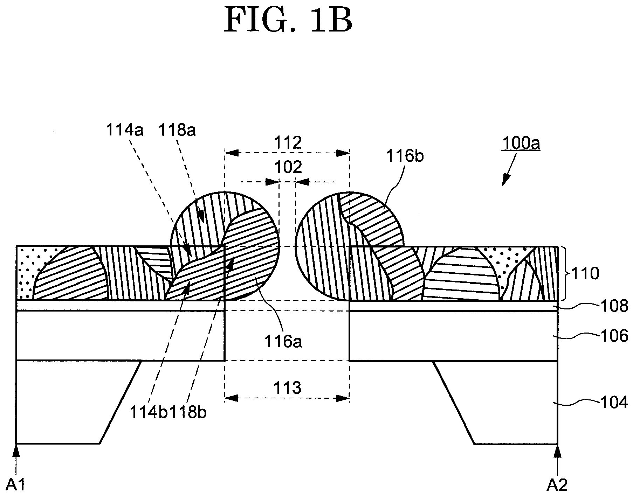

FIG. 1B shows the cross-sectional structure corresponding to the line A1-A2 shown in FIG. 1A. The nanopore structure 100a shown in FIG. 1B has a structure in which a first metal member 110 is formed on an insulating layer 106 and the insulating layer 106 is supported by a support member 104. With respect to the through hole 112 formed in the first metal member 110, a through hole 113 overlapping the through hole 112 and having substantially the same hole diameter is formed in the insulating layer 106. The support member 104 is formed in contact with the insulating layer 106 at a position where it does not overlap the through hole 113.

As shown in FIG. 1B, a base metal layer 108 may be formed between the first metal member 110 and the insulating layer 106. Although the base metal layer 108 is not an essential structure in the nanopore structure 100a, it is formed as necessary so that the first metal member 110 is stably supported on the insulating layer 106 (i.e., to enhance adhesion).

FIG. 1B shows a configuration in which the second metal member 116a is formed at one end of the through hole 112 and the second metal member 116b is formed at the other end facing the one end when the nanopore structure 100a is viewed in a cross section. The second metal members 116a, 116b have a nanoscale island structure, a mountain shape in appearance, and a spherical surface. The second metal members 116a, 116b having such a shape are formed to contact the side wall (side surface of the first metal member 110) of the through hole 112 from the upper surface of the first metal member 110. Here, the spherical surface is defined as a curved surface having continuous curved surfaces while the radius of curvature continuously changes, and is not limited to the spherical surface.

The nanopore structure 100a is formed of metal materials in which the first metal member 110 and the second metal members 116a, 116b are different from each other. As a suitable metal material for forming the first metal member 110, transition elements such as platinum (Pt), palladium (Pd), rhodium (Rd), ruthenium (Ru), osmium (Os), and iridium (Ir) are exemplified. As a suitable metal material for forming the second metal members 116a, 116b, gold (Au) is exemplified.

Although the method of manufacturing the nanopore structure 100a will be described later, at least the second metal members 116a, 16b are formed by an electroless plating method. The electroless plating method enables the second metal members 116a, 116b having a size of nanoscale to be selectively formed on the surface of the first metal member 110. That is, the electroless plating method makes it possible to provide the second metal members 116a, 116b on the surface of the first metal member 110 without adhering to the surfaces of the insulating layer 106 and the base metal layer 108 and to narrow the hole diameter of the through hole 112.

The first metal member 110 and the second metal members 116a, 116b may have a crystal structure. FIG. 1B schematically illustrates an embodiment in which the first metal member 110 has a polycrystalline structure and includes crystal grains 114a, 114b, and the second metal member 116a includes crystalline regions 118a, 118b. The crystalline region 118a corresponds to the crystalline grain 114a, and the crystalline region 118b corresponds to the crystalline grain 114b. In this case, although the first metal member 110 and the second metal members 116a, 116b are different metals, the crystalline regions 118a, 118b are preferably formed as regions heteroepitaxially grown from the crystal grains 114a, 114b, respectively.

It is necessary for the crystal regions 118a, 118b to be lattice-matched in order to heteroepitaxially grow from the crystal grains 114a, 114b. More specifically, it is desirable that the ratio of lattice mismatch between the lattice constant of the first metal member 110 and the lattice constant of the second metal members 116a, 116b is 35% or less, preferably 10% or less, and more preferably 5% or less.

For example, the lattice constant of platinum (Pt) exemplified as the first metal member 110 is 0.39242 nm, that of palladium (Pd) is 0.38907 nm, and that of gold (Au) exemplified as the second metal member 116 is 0.40782 nm. Since the ratio (misfit ratio) of lattice mismatch between platinum (Pt), palladium (Pd) and gold (Au) is 3.9% and 4.6%, respectively, it is possible to heteroepitaxially grow the second metal member 116 formed of gold (Au) on the surface of crystal grains 114 formed of platinum (Pt) or palladium (Pd).

The second metal members 116a, 116b including a crystalline region heteroepitaxially grown from the first metal member 110 have a nanoscale island structure. The second metal members 116a, 116b have a spherical shape in a cross-sectional view. The size of the second metal members 116a, 116b having a nanoscale island-like structure has a width from one end to the other end of 50 nm or less, preferably 20 nm or less, more preferably 10 nm or less in a plan view (when the first metal member 110 is viewed from the upper surface). The second metal members 116a, 116b have a height of 40 nm or less, preferably 20 nm or less, more preferably 10 nm or less from the surface of the first metal member 110.

In the present invention, the nanoscale island structure refers to an individual body having a size of about 50 nm or less. The second metal members 116a, 116b are heteroepitaxially grown in a highly wettable condition to form spherical projections. Since the electroless plating does not proceed on the surface of the base metal layer 108, the end of the spherical projection of the second metal member having the nanoscale is located at the boundary between the first metal member 110 and the base metal layer 108. Therefore, the angle of the spherical projection of the second metal member can be defined by a straight line extending into the hole from the interface between the first metal member 110 and the base metal film 108. The positive angle means the spherical protrusion of the second metal member does not protrude toward the base metal layer 108, and the negative angle means the spherical protrusion protrudes toward the base metal layer 108. The angle is preferably positive, but may be negative.

The second metal members 116a, 116b include crystal regions having different crystal orientations and are circular in a cross section. This is because the surface tension, which is proportional to the inverse of the radius of curvature, becomes extremely large when the radius of curvature is 15 nm or less, and the atoms constituting the second metal members 116a, 116b have a circular cross-section so that the surface self-diffuses and minimizes the surface energy. When the second metal members 116a, 116b are viewed in a cross section, their circular shape has a structure in which a part of a circle passes through the interface between the base metal layer 108 and the first metal member 110.

When the first metal member 110 is platinum (Pt) and the second metal members 116a, 116b are gold (Au), if the radius of curvature of the gold (Au) becomes small, the gold (Au) becomes spherical which reduces the surface energy. Platinum (Pt) is harder and has a much smaller surface self-diffusion coefficient than gold (Au). Therefore, gold (Au) grown heteroepitaxially on platinum (Pt) is in contact with platinum (Pt), gold (Au) atoms are pinned by platinum (Pt) atoms and become less mobile. As a result, the gold (Au) particles are fixed in contact with the platinum (Pt) and become spherical with a small radius of curvature which reduces the surface energy.

This condition means that the second metal members 116a, 116b formed to narrow the hole diameter of the through hole 112 can stably maintain the shape of the nanopore 102. For example, as shown in FIG. 1A, it is possible to maintain the structure of the nanopore 102 while keeping the shape of the hole circular by surrounding the periphery of the through hole 112 with the second metal member 116 having a spherical surface.

Further, when the radius of curvature of the second metal members 116a, 116b is smaller, the number of the nearest metal atoms adjacent to the sample becomes one in the nanopore 102, and its size is smaller than, for example, 0.36 nm, which is the distance between bases of DNA, so that the spatial resolution can be improved, and as described later, when a current flows from the DNA to the nanopore 102 by a tunnel current, the current flowing for each base can be read.

When the radius of curvature of the second metal members 116a, 116b is equivalent to the length of the cross section of the DNA, the ion current flowing between the trans chamber and the cis chamber through the nanopore is strongly affected by the base, and the base species reading accuracy is improved.

The nanopore structure 100a needs to precisely control the pore diameter of the nanopore 102. As described above, the second metal members 116a, 116b are formed by a chemical film deposition method by electroless plating. As described above, it is considered that the controllability of the electroless plating may become a problem when forming the nanopore 102 by the constriction of the second metal members 116a, 116b.

If the growth of the second metal members 116a, 116b continues indefinitely by electroless plating, the through hole 112 is closed, and the reaction must be controlled by time. In this case, there is concern that the variation between manufacturing will increase.

However, according to the electroless plating described in detail in this embodiment, the growth of the second metal members 116a, 116b does not continue indefinitely, but stops automatically. As shown in FIG. 1B, a Helmholtz layer (a layer of solvent, solute molecules and solute ions adsorbed on the electrode surface) is formed when the distance between the second metal member 116a and the second metal member 116b is narrowed. When the Helmholtz layer is formed, metal ions in the electroless plating solution cannot enter the gap. As a result, plating growth in the opposite regions of the second metal member 116a and the second metal member 116b is automatically stopped.

The second metal members 116a, 116b grow selectively on the surface of the first metal member 110 and not on other portions (e.g., the base metal layer 108) in the electroless plating process. The open ends of the through holes 112 formed in the first metal member 110 are subject to electric field concentration in the electroless plating solution, which increases the nucleation probability, ensuring the growth of the second metal members 116a, 116b. In this case, the thickness of the first metal member 110 affects the radius of curvature of the second metal members 116a, 116b.

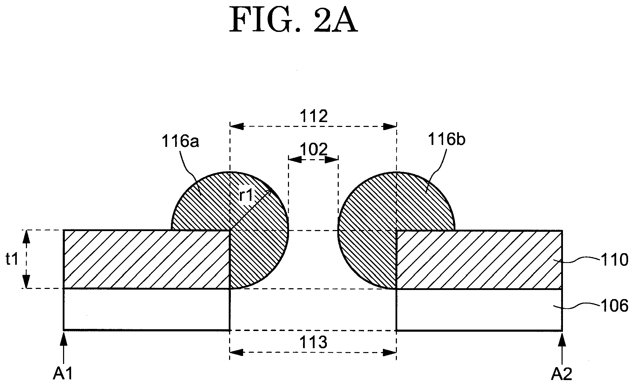

For example, as shown in FIG. 2A, when a thickness of the first metal member 110 is t1 and a radius of curvature of the second metal member 116a is r1, the angle becomes 0 degrees when t1 becomes equal to r1. To further progress the plating and create a state where t1 < r1, the internal stress of the second metal member 116 is increased, so the progress of plating is slower than in the case where t1 > r1. Therefore, the radius of curvature r1, r2 of the second metal members 116a, 116b can be controlled to be equal to the thickness t1 of the first metal member 110. When the plating time is extended, the plating is further advanced, so that a state of t1 < r1 can be formed.

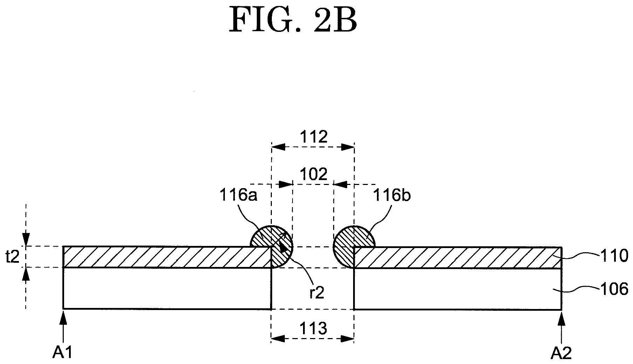

When the film thickness of the first metal member 110 is set to t2 smaller than t1 as shown in FIG. 2B, the radius of curvature of the second metal member 116a is reduced from r1 to r2 by selecting a plating condition. According to this embodiment, the radius of curvature of the second metal member 116a can be controlled by adjusting the thickness of the first metal member 110.

As described above, the pore diameter of the nanopore 102 can be controlled by two methods, that is, the automatic stop of the plating described above and the control by the combination of the film thickness t1 of the first metal member 110 and the initial pore diameter.

The thickness of the first metal member 110 may be 20 nm or less, preferably 10 nm or less, more preferably 5 nm or less, more preferably 3 nm or less. However, it is preferable that the diameter of the through hole 112 (the length of one side of a square or the diameter of a circular hole) is substantially equal to the thickness of the first metal member 110 because the reduction width of the hole diameter by electroless plating is limited when the first metal member 110 is made thin.

The radius of curvature by the thickness t1 of the first metal member 110 may cause r1 to exceed t1 as plating begins to progress again as the plating time is further increased. In this case, the self-stopping function due to the ionic radius is also effective.

The spherical cross-sectional shape of the second metal member 116 is due to growth on the surface of the first metal member 110 by electroless plating. For example, the physical deposition method, such as the oblique evaporation method, results in a structure that does not have a radius of curvature because the areas that shadows are uniformly deposited and thus the thickness of the deposited layer is uniform and is deposited regardless of the boundary between the first metal member 110 and the base metal layer 108. Further, the oblique evaporation method causes to form a layer not only on the first metal member 110 but also on the base metal layer 108 and the insulating layer 106.

According to the present embodiment, the pore diameter of the nanopore 102 can be precisely controlled by utilizing the self-stopping function developed by the electroless plating. In other words, the pore diameter of the nanopore 102 is controlled not by a microfabrication technique such as photolithography and etching but by two reaction rate determine of electroless plating, so that the pore diameter of 10 nm or less can be precisely controlled with good reproducibility.

The materials of the support member 104, the insulating layer 106, and the base metal layer 108 shown in FIG. 1B are not limited. For example, a silicon substrate (silicon wafer) which is thermally stable can be used as the support member 104. An inorganic insulating material such as a silicon oxide, a silicon nitride or an aluminum oxide can be used as the insulating layer 106, and a metal material such as titanium (Ti), molybdenum (Mo), tantalum (Ta) or chromium (Cr) can be used as the base metal layer 108.

As described above, the nanopore structure 100a according to the present embodiment can obtain the nanopore 102 having a pore diameter of 10 nm or less by providing the second metal member 116 having one or more nanoscale island structures so as to narrow the pore diameter of the through hole 112 formed in the first metal member 110.

### 1-2. Second Embodiment

The second embodiment has a structure in which the second metal member 116 is continuous over the first metal member 110, while the first embodiment has a nanoscale island structure of the second metal member 116.

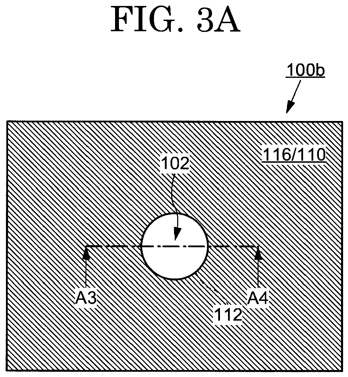

FIG. 3A shows a plan view of the nanopore structure 100b. The nanopore structure 100b differs from the first embodiment in that the second metal member 116 has a continuous structure on the first metal member 110. The nanopore structure 100b is similar to the nanopore structure 100a according to the first embodiment in terms of other members including the pore diameter of the nanopore 102.

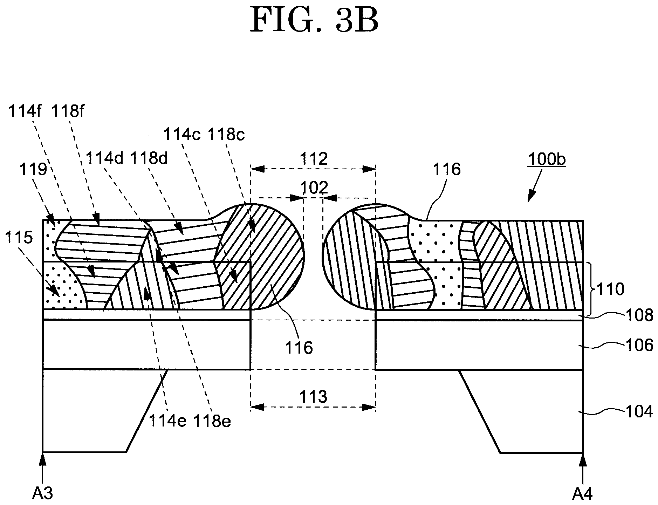

FIG. 3B shows the cross-sectional structure corresponding to the line A3-A4 shown in FIG. 3A. The metal materials forming the first metal member 110 and the second metal member 116 are the same as those shown in the first embodiment. The first metal member 110 has a polycrystalline structure and includes a plurality of crystal grains 114. FIG. 3B schematically shows a configuration in which the first metal member 110 includes crystal grains 114c, 114d, 114e, 114f.

The second metal member 116 includes crystal regions 118b, 118c, 118d, 118e heteroepitaxially grown corresponding to the crystal grains 114b, 114c, 114d, 114e. The second metal member 116 having such crystallinity is formed by electroless plating.

The first metal member 110 may also include an amorphous region 115. Since nucleation also occurs in the amorphous region 115 during the electroless plating, the second metal member 116 also grows in that region. The second metal member 116 growing on the amorphous region 115 is an amorphous (amorphous region 119). When the second metal member 116 is heteroepitaxially grown on the first metal member 110, lattice strain or the like may occur during the growth, and an amorphous region may be included. Thus, the second metal member 116 may include a plurality of heteroepitaxially grown crystalline regions 118b, 118c, 118d, 118e, and even amorphous regions 115.

Since the first metal member 110 and the second metal member 116 include a heteroepitaxially grown interface, crystal continuity is maintained between different metals, and the thermal stability of the structure is high. Therefore, the shape of the nanopore structure 100a can be kept stable.

As shown in FIG. 3B, the second metal member 116 is formed to continuously surround the open end of the through hole 112 of the first metal member 110. The second metal member 116 extends from the upper surface of the first metal member 110 to the side wall of the through hole 112, and is disposed to protrude into the hole portion of the through hole 112. The second metal member 116 protrudes into the hole portion of the through hole 112 so as to narrow the hole diameter, as in the first embodiment.

The second metal member 116 is formed on the first metal member 110 by electroless plating. The second metal member 116 has a continuous structure in the second embodiment, while the first embodiment has a spherical island structure. These structural differences can be controlled by electroless plating conditions and / or pretreatment conditions. For example, it is possible to form the second metal member 116 having a continuous film-like structure by electroless plating by changing the surface state of the first metal member 110 by pretreatment to increase the nucleation density.

As shown in FIG. 3A and FIG. 3B, the second metal member 116 epitaxially grown from the first metal member 110 is connected to continuously cover the end portion of the through hole 112 while including the grain boundary. The nanopore 102 has a surface in which crystal grains having a predetermined radius of curvature are connected to form a circumference by a continuous curved surface. The inner surface of the nanopore 102 has a circumferential structure with connected spherical surfaces because the surface tension increases in proportion to the inverse of the radius.

As described above, the nanopore structure 100b according to the second embodiment can obtain the nanopore 102 having a size of 10 nm or less, preferably 1 nm to 5 nm by providing the second metal member 116 having a continuous structure to narrow the hole diameter of the through hole 112 formed in the first metal member 110. The second metal member 116 continuously surrounds the through hole 112 of the first metal member 110 in the second embodiment, so that the shape of the nanopore 102 can be made to be nearly circular. Even if the shape of the through hole 112 is square in a plan view, it can be formed into a shape close to a circle by surrounding it with the second metal member 116. As a result, the variation between individual bodies with respect to the shape and pore diameter of the nanopore 102 can be reduced.

The shape of the second metal member 116 forming the nanopore 102 can have several shapes depending on electroless plating and / or pretreatment conditions. If the nucleation density is higher in the open end region of the through hole 112 than in the flat portion, the circular portion of the second metal member 116 is higher in the portion surrounding the through hole 112 than in the other flat portion of the second metal member 116 (the flat portion on the first metal member 110). Further, the circular portion of the second metal member 116 covers only the first metal member 110 as in the first embodiment, and the second metal member 116 does not grow on the surface of the base metal layer 108.

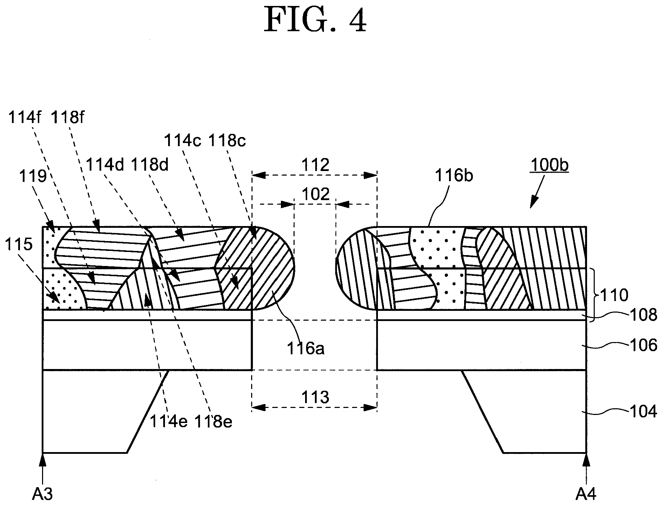

On the other hand, as shown in FIG. 4, the second metal member 116 may grow to a region surrounding the through hole 112 at the same height as the flat portion, and the crystal region 118c at the tip portion may form a spherical surface having a predetermined radius of curvature. These shape differences can be made separately by controlling the surface conditions of the first metal member 110 and the open ends of the through holes 112, which serve as the base surfaces during electroless plating (that is, by controlling the nucleation density).

### 1-3. Third Embodiment

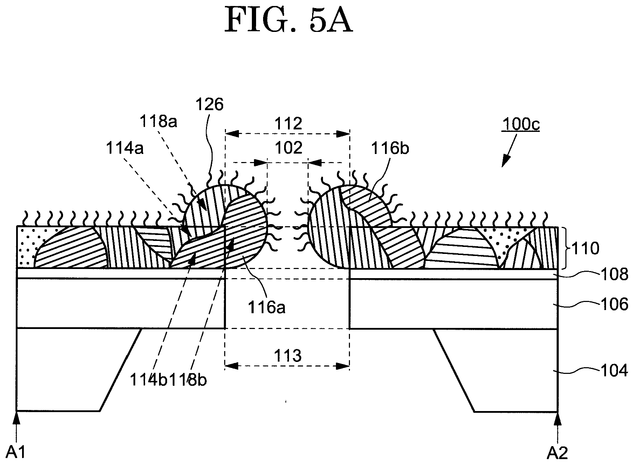

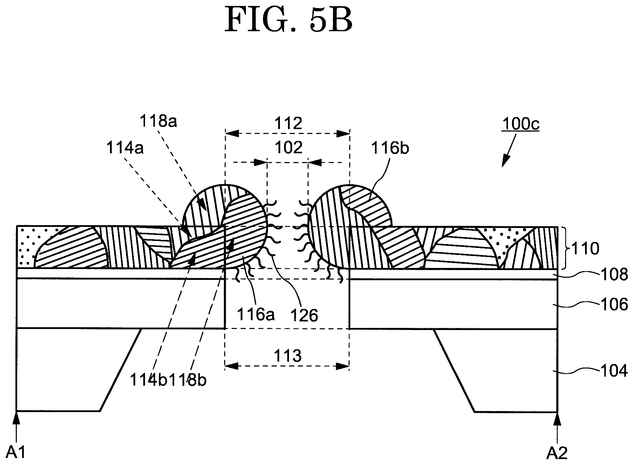

It is also possible to provide a self-assembled monolayer on the surface of the second metal member 116 in the nanopore structures shown in the first and second embodiments. FIG. 5A and FIG. 5B show an example of a nanopore structure 100c in which a self-assembled monolayer (SAM) is arranged on the surface of the second metal members 116a, 116b in the first embodiment.

The self-assembled monolayer 126 includes a first functional group chemisorbed on the second metal members 116a, 116b and a second functional group bonded to the first functional group. The first functional group is one of a thiol group, a dithiocarbamate group and a xanthate group. The second functional group is an alkane, an alkene, an alkane or a group obtained by substituting a part or all of a hydrogen molecule of the alkene with fluorine, an amino group, a nitro group, an amide group, and any group containing the following fluorescent dye.

For example, the self-assembled monolayer 126 is formed of a monolayer obtained by self-assembling an alkanethiol. The self-assembled monolayer 126 is water repellent and acts to keep the surface condition. A small number of alkane dithiols are mixed in the alkane thiols of the self-assembled monolayer 126. The alkane dithiol is a structure in which a thiol is positioned at a bonding group containing sulfur (S) at both ends of an alkane chain, and sulfur (S) exists at locations in an alkane thiol monolayer. In order to mix an alkane dithiol in an alkane thiol, an electrode coated with an alkanethiol self-assembled monolayer 126 is immersed in a solution of the alkane dithiol, and a part of the alkane thiol is replaced with the alkane dithiol.

The self-assembled monolayer 126 has a length of 1 nm to 5 nm. The diameter of the nanopore 102 can be further narrow by coating the self-assembled monolayer 126 having such a length on the surfaces of the second metal members 116a, 116b. The self-assembled monolayer 126 may be formed in a portion that seeps beyond the narrowest portion of the nanopore 102 that can be viewed from the side of the support member 104 when immersed in a solution containing the self-assembled monolayer material by controlling the preparation conditions, or may not be formed to the narrowest portion of the nanopore 102.

The self-assembled monolayer is formed on the surfaces of the second metal members 116a, 116b and the first metal member 110 when they are immersed in a solution containing molecules forming the self-assembled monolayer. Therefore, when the first metal member 110 and the second metal members 116a, 116b are immersed in a solution containing the self-assembled monolayer material only on either side of the support member 104, it can control the surface on which the self-assembled monolayer is formed. That is, as shown in FIG. 5A, when immersed only on the side where the first metal member 110 and the second metal members 116a, 116b are disposed, a self-assembled monolayer is formed on the second metal members 116a, 116b forming the nanopore and the surface of first metal member. On the other hand, as shown in FIG. 5B, when immersed in a solution containing the self-assembled monolayer material only on the side of the support member 104, the self-assembled monolayer can be formed only on the nanopore portion which can be seen from the side of the support member 104. Thus, when the self-assembled monolayer is formed on only one side, the opposite side may be a solvent or a solution not containing the self-assembled monolayer material, or the opposite side may not be immersed in the solution.

It is possible to create a situation in which the self-assembled monolayer 126 is formed on the surfaces of the second metal members 116a, 116b including the upper surface of the narrowing portion of the nanopore and the surface of the first metal member 110, and a situation in which the self-assembled monolayer 126 is formed only on the lower surface of the narrowing portion of the nanopore and the surface of the narrowing portion.

It is also possible to form a self-assembled monolayer 126 on the entire surface of the second metal member 116 and the entire surface of the first metal member 110 when the entire structure is immersed in a solution containing a self-assembled monolayer material.

The self-assembled monolayer 126 may include phosphor molecules that label specific bases contained in DNA and RNA. For example, different phosphors can be included in each of the self-assembled monolayers 126 so that different phosphors are associated with each of the four bases (adenine, guanine, thymine, and cytosine) included in the DNA. It is possible to perform fluorescence analysis when decoding the base sequence of a sample (DNA, RNA, etc.) as a time-series optical signal by using the nanopore 102 by providing the self-assembled monolayer 126 having such a fluorescent dye.

It is preferable that the self-assembled monolayer 126 is provided only on the narrowed lower surface and the narrowed surface of the nanopore on the side of the through hole 113 of the insulating layer 106 when the self-assembled monolayer containing the fluorescent dye is formed. Thus, a self-assembled monolayer containing a fluorescent dye is coated only in the nanopore portion on the cis chamber side.

For example, a fluorescein dye, a rhodamine dye, a xanthene dye, or the like can be used as the phosphor intercalating with DNA or RNA.

When performing the fluorescence analysis, the excitation light is preferably irradiated from the trans-chamber side, but may be irradiated from the cis-chamber side. The self-assembled monolayer is formed so that the fluorescent dye is located only in the portion of the nanopore 102, and the fluorescence that changes in combination with the base of the DNA can be detected with a high SN ratio. The fluorescence is preferably detected from the incident side by using an optical system for irradiating excitation light and a half mirror. The portion of the nanopore 102 contains gold (Au), and the surface of the gold (Au) amplifies the excitation light intensity by surface plasmon enhancement. Thus, photons of the excitation light can efficiently excite the fluorescent dye. When the excitation light is detected, there is a case where the excitation light is quenched when a metal exists in the vicinity of the dye. This quenching can be suppressed by appropriately controlling the distance between the dye and the nanopore by the self-assembled monolayer.

The self-assembled monolayer 126 is 0.3 nm to 5 nm in length, so the effective pore size of the nanopore 102 is reduced by that length. It is also preferable because the affinity between the self-assembled monolayer 126 and the DNA base prevents quenching between the second metal member 116 and the fluorescent dye, compared to the metal surface of the nanopore 102.

### 1-4. Fourth Embodiment

The nanopore according to this embodiment is formed of a metallic material. Therefore, nanopores can be formed in each of a plurality of electrodes (electrically separated electrodes).

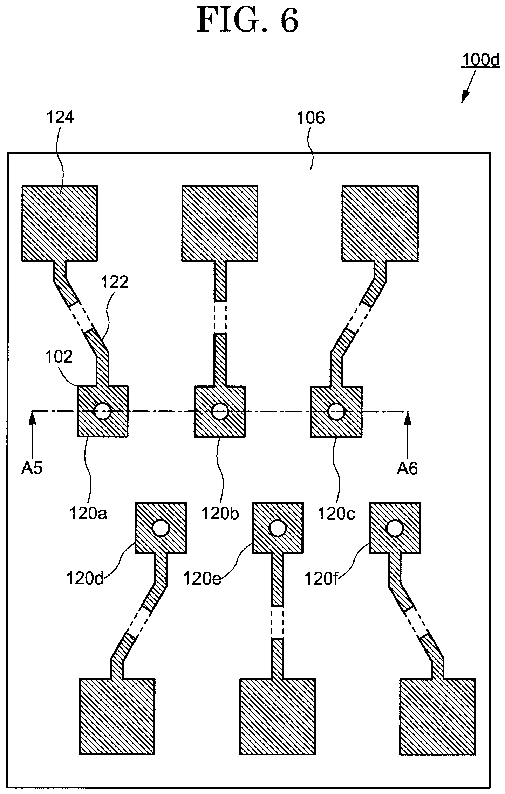

FIG. 6 shows a plan view of the nanopore structure 100d according to the fourth embodiment. The nanopore structure 100d has a plurality of electrodes including nanopores 102 are arranged on an insulating surface. FIG. 6 illustrates, as an example, an arrangement of six nanopore electrodes 120a-120f. The electrode 120a with the nanopore is formed with the nanopore 102 at a substantially central portion, and is connected to an electrode pad 124 formed at a peripheral portion of the nanopore structure 100d by a wiring 122. Other nanopores electrodes 120b to 120f have similar configurations.

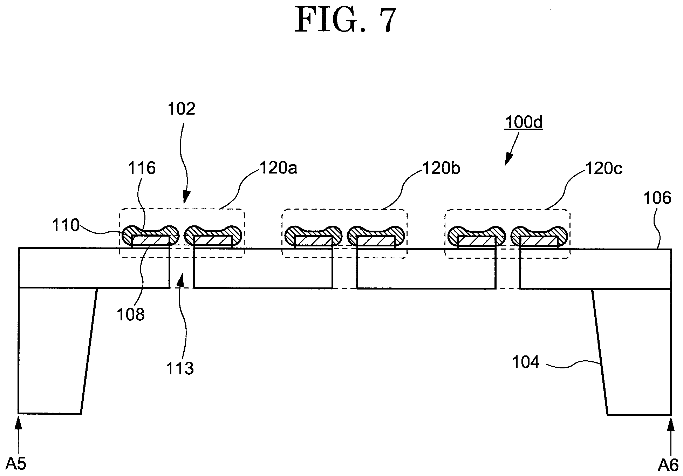

FIG. 7 shows the cross-sectional structure corresponding to the line A5-A6 shown in FIG. 6. The nanopore electrode 120a is formed of the first metal member 110 and the second metal member 116 arranged on the insulating layer 106. The first metal member 110 and the second metal member 116 forming the nanopore 102 are the same as those described in the first to third embodiments. Although not shown in FIG. 7, the wiring 122 and the electrode pad 124 may be formed of the same metal material as that of the first metal member 110, and the second metal member 116 may be formed on the surface thereof.

It is possible to pattern a plurality of regions on the insulating layer 106 since the first metal member 110 is formed of a thin metal layer, and thereby, the electrode 120 with the nanopore provided with the nanopore 102 can be integrated. It is possible to pattern a plurality of regions on the insulating layer 106 since the first metal member 110 is formed of a thin metal layer, and thereby, the electrode 120 provided with the nanopore 102 can be integrated. The nanopore structure 100c in which the electrode 120 in which the nanopore 102 is formed is integrated can be used for a DNA sequencer as described later. The electrode 120 provided with the nanopore 102 controls the potential independently in the DNA sequencer and can be used for the application of DNA decoding.

When the through hole 113 of the insulating layer 106 is used as a microchannel for untangling the clump of single-stranded DNA, the lower part of FIG. 7 is a cis chamber and the upper part is a trans chamber, which can be used by inverting the upper and lower parts.

### 1-5. Method for Manufacturing Nanopore Structure

This embodiment shows a method of manufacturing by combining electroless gold plating (ELGP) and electron beam lithography (EBL).

A silicon substrate (silicon wafer) 130 formed insulating layer 131, 132 on the surface thereof is used. The silicon substrate 130 is preferably polished on both sides. The insulating layers 131, 132 are, for example, silicon oxide layers formed by thermally oxidizing the silicon substrate 130. The thickness of the silicon substrate 130 is about 300 µm to 600 µm, and the thickness of the insulating layers 131, 132 is 100 nm to 500 nm, for example, 300 nm.

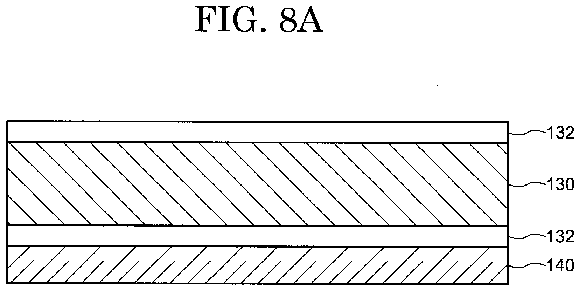

FIG. 8A shows a step of forming a photoresist 140 on the back surface side of the silicon substrate 130. The electrode provided with the nanopore is formed on the front surface side of the silicon substrate 130 (that is, on the side where the insulating layer 132 is disposed), so that the photoresist 140 may also be formed on the front surface of the insulating layer 132.

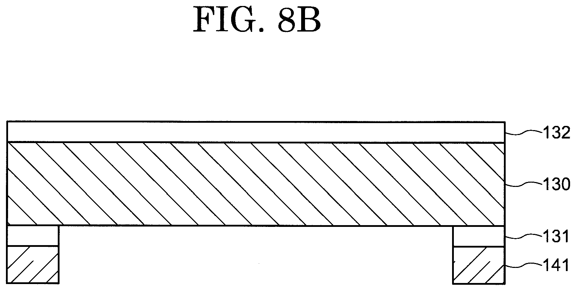

FIG. 8B shows a step of exposing and developing the photoresist 140, forming a resist mask 141, and etching the insulating layer 131 on the back surface. Wet etching using hydrofluoric acid or the like or reactive ion etching (RIE) is performed. The size of the pattern of the opening of the resist mask 141 is arbitrary, but, for example, the length of one side is formed to a size of 100 µm to 500 µm.

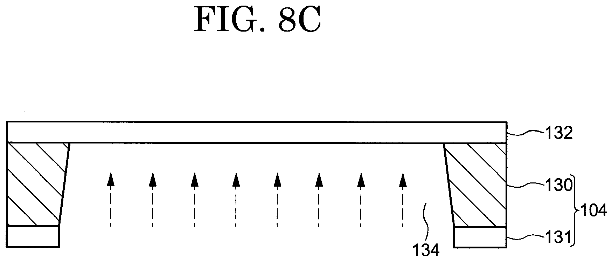

FIG. 8C shows a step of etching the silicon substrate 130 from the back surface by anisotropic etching to expose the insulating layer 132 on the front surface side. For example, anisotropic etching is performed for the silicon substrate 130 by inductively coupled plasma reactive ion etching (ICP-RIE) using a CF₄-O₂ based or SF₆-O₂ based etching gas. Further, the silicon substrate 130 may be subjected to wet etching with an alkaline aqueous solution such as KOH (potassium hydroxide), TMAH (tetramethylammonium hydroxide) or EDP (ethylenediamine pyrocatel) to a certain depth, and then subjected to anisotropic etching. The insulating film 131 left on the back surface of the silicon substrate 130 is used as a hard mask in this etching.

As a result of the anisotropic etching, an opening 134 is formed in the silicon substrate 130 so that the insulating layer 132 is exposed on the back surface side. The silicon substrate 130 and the insulating layer 131 surrounding the opening 134 are used as the support member 104.

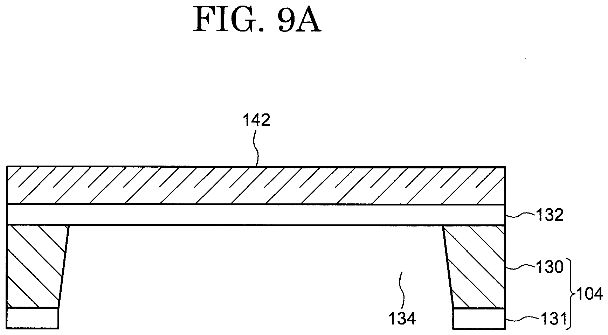

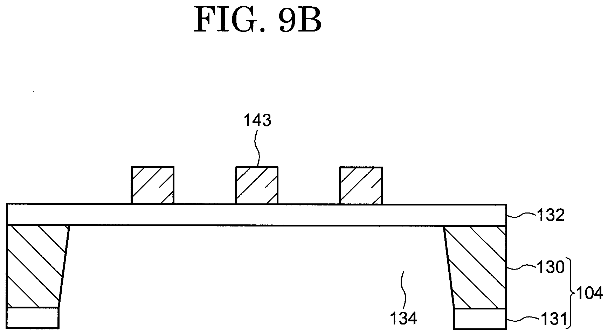

Next, as shown in FIG. 9A, a photoresist 142 is formed on the upper surface of the insulating layer 132. Then, the photoresist is exposed to light to form a resist pattern 143 as shown in FIG. 9B. For example, a positive resist for electron beam exposure is used, and is exposed using an electron beam lithography system, thereby forming a resist pattern 143. Nanopores are formed at an area of the resist pattern 143. The planar shape of the resist pattern 143 may be circular or square.

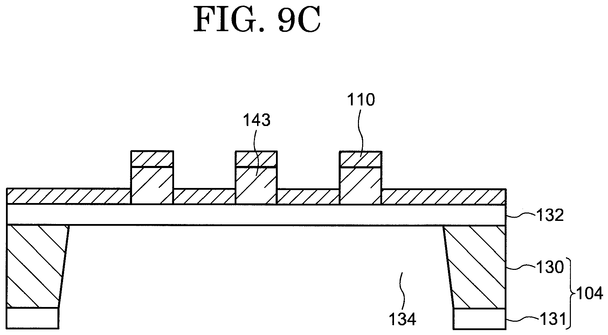

As shown in FIG. 9C, a metal layer which becomes the first metal member 110 is formed on the insulating layer 132 on which the resist pattern 143 is formed. The metal layer is formed by electron beam evaporation or sputtering. The metal layer may have a two-layer structure. For example, it may have a two-layer structure of a titanium (Ti) layer and a platinum (Pt) layer. The metal layer is formed with a thickness of 3 nm to 20 nm. For example, a platinum (Pt) layer of 40 nm or less, preferably 20 nm or less, more preferably 10 nm or less, and 3 nm or more is formed on a titanium (Ti) layer of 1 nm to 3 nm.

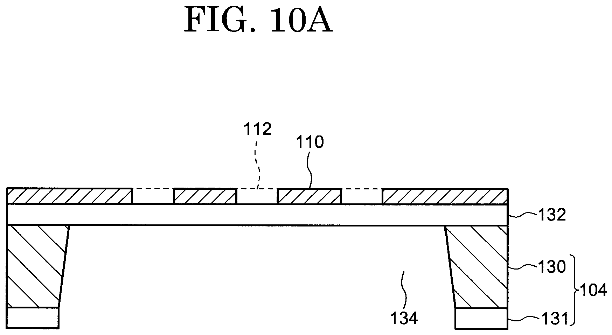

Then, as shown in FIG. 10A, when the resist is removed by the lift-off process, a through hole 112 is formed in the first metal member 110, and the insulating layer 132 is exposed. Although not shown in FIG. 10A, at this step, the metal layer may be patterned to form electrode pads and wirings.

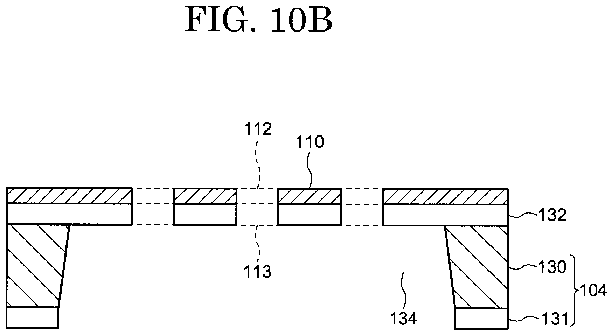

Next, as shown in FIG. 10B, a through hole 113 is formed in the insulating layer 132. The insulating layer 132 is etched by reactive ion etching using the first metal member 110 patterned by lift-off as a mask to form the through hole 113. The insulating layer 132 and the through hole 113 shown in FIG. 10B correspond to the insulating layer 106 and the through hole 113 shown in FIG. 1B and FIG. 3B.

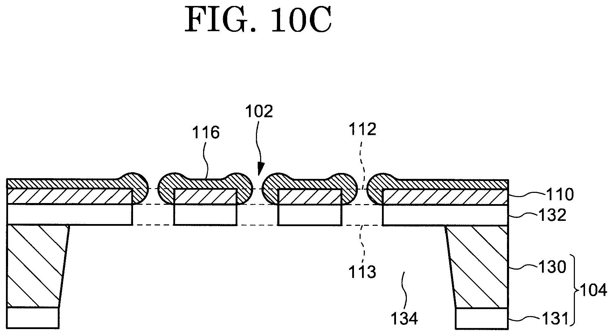

As shown in FIG. 10C, the second metal member 116 is formed so as to cover the surface of the first metal member 110. The second metal member 116 is formed by electroless plating. The second metal member 116 is formed to cover the end of the opening of the through hole 113 and narrow the hole diameter. The nanopore structure 100 having the nanopore 102 can be manufactured by such a process.

The step of removing the insulating layer 131 and the silicon substrate 130 to form the opening 134 shown in FIG. 8B and FIG. 8C may be performed after the nanopore 102 is formed.

Alternatively, instead of forming the resist pattern 143 shown in FIG. 9B, a metal layer for forming the first metal member 110 may be deposited on the entire surface of the insulating layer 132, and the metal layer and the insulating layer 132 may be partially removed by using a focused ion beam (FIB) to form the structure shown in FIG. 10B.

The pore diameter of the nanopore 102 is controlled not only by processing the first metal member 110 by photolithography and etching but also by forming the second metal member 116 by electroless plating. Next, the electroless plating will be described in detail.

### 1-6. Electroless Plating

Electroless plating for forming the second metal member will be described below.

### 1-6-1. Electroless Plating Solution

A solution containing gold ions (Au⁺, Au³⁺), halogen ions as an oxidizing agent and a reducing agent are used as the electroless gold plating solution. In order to heteroepitaxially grow gold (Au) on platinum (Pt) by electroless gold plating, it is necessary to reduce platinum oxide (PtO) existing on the surface of platinum (Pt). The electroless plating solution is appropriately selected as a combination of halogen element ions and a reducing agent in order to express this reducing action. Further, gold (Au) is deposited by controlling the ratio of the reduction reaction by excessively containing a reducing agent. Furthermore, the electroless gold plating solution is diluted with a large amount of pure water to control the gold (Au) reduction rate so that no gold (Au) particles are deposited in the electroless plating solution.

As the electroless plating solution in the present embodiment, an electroless gold plating solution in which iodine tincture in which gold (Au) is dissolved and L(+)-ascorbic acid (C₆H₈O₆) used as a reducing agent are combined is used. This electroless gold plating solution allows heteroepitaxial growth of gold (Au) on the platinum (Pt) crystal surface. It is considered that the electroless gold plating solution contains iodine ions (I⁻, I₃⁻) derived from iodine tincture and L(+)-ascorbic acid (C₆H₈O₆), thereby causing a reduction reaction of platinum oxide (PtO or PtO₂).

### 1-6-2. Method for Electroless Gold Plating

The electroless plating is performed by immersing a platinum (Pt) as the first metal member 110 in an electroless gold plating solution. When a platinum (Pt) as the first metal member 110 is immersed in an electroless gold plating solution, nuclei are preferentially formed on the surface of crystal grains of the platinum (Pt), and gold (Au) reduced from gold ions (Au⁺, Au³⁺) grows. The electroless gold plating solution is diluted 100 times, preferably 500 times or more, more preferably 1000 times or more with pure water as described above. The electroless plating solution contains an excessive amount of a reducing agent.

The electroless gold plating solution according to the present embodiment contains an excess of a reducing agent in the solution before dilution, so that gold (Au) ions are reduced from trivalent gold ions (Au³⁺) to monovalent gold ions (Au⁺).

The reduction potential (Reference standard hydrogen electrode, 25°C, 105 Pa) from a monovalent gold ion (Au⁺) to gold (Au) or from a trivalent gold ion (Au³⁺) to a monovalent gold ion (Au⁺) are as follows.

Au⁺ + e⁻ → Au: 1.82 V

Au³⁺ + 2e⁻ → Au⁺: 1.41 V

Au³⁺ + 3e⁻ → Au: 1.52 V

The reduction potential from platinum ions (Pt) to platinum (Pt) is as follows.

Pt²⁺ + 2e⁻ → Pt: 1.188 V

When the trivalent gold ion (Au³⁺) is reduced to the monovalent gold ion (Au⁺), the reduction potential to gold (Au) increases, and the monovalent gold ion (Au⁺) is reduced less than the trivalent gold ion (Au³⁺). The dilution with pure water shifts the equilibrium of iodine (I₂), which is difficult to dissolve in pure water, to iodine ions (I⁻, I₃⁻), and the ratio of iodine ions to iodine increases. Since the iodine ion has an etching gold (Au) action, an excess of the reducing agent has an effect of suppressing etching. Compared with the reduction potential (1.188 V) of the platinum ion (Pt²⁺), the reduction potentials of the trivalent gold ion (Au³⁺) and the monovalent gold ion (Au⁺) are as high as 1.52 V and 1.82 V, respectively, and are difficult to reduce. The excess reducing agent is effective for promoting the electrochemical substitution reaction of platinum and gold by its reducing action.

The electroless gold plating solution according to the present embodiment is diluted 100 times or more, preferably 500 times or more, with pure water, so that gold (Au) can be heteroepitaxially grown on the first metal member such as platinum (Pt). When the dilution ratio is small, the growth rate of the electroless gold plating becomes fast, the heteroepitaxial growth becomes impossible, the nuclei are formed in the plating bath and grow as gold nanoparticles, and the possibility of physical adsorption of the gold nanoparticles on the surface of the first metal member increases. A growth rate that enables heteroepitaxial growth of gold (Au) is obtained when diluted 1000 times. Therefore, in order to heteroepitaxially grow gold (Au), the dilution ratio of pure water is important in order to control the growth rate of plating at the dilution ratio as described above.

The platinum (Pt) layer or palladium (Pd) layer as the first metal member 110 immersed in the electroless plating solution is removed from the electroless plating solution before gold ions (Au⁺, Au³⁺) are reduced and deposited in the electroless gold plating solution and deposited on the surface of the first metal member 110 while heteroepitaxially growing gold (Au) on the surface as described above. This processing is repeated at least once, preferably a plurality of times to form a region of gold (Au) as the second metal member 116. The immersion time is appropriately set according to the concentration and temperature of the electroless gold plating solution. For example, the time for immersing the first metal member 110 in the electroless gold plating solution is controlled from 3 seconds to 30 seconds, for example, 10 seconds.

Specifically, the electroless gold plating solution in which an iodine tincture in which gold (Au) is dissolved and L(+)-ascorbic acid (C₆H₈O₆) used as a reducing agent are combined produces a condition in which gold (Au) can be heteroepitaxially grown on a platinum (Pt) surface by reducing a platinum oxide (PtO) formed on the surface of a platinum (Pt) layer by a reduction reaction expressed by a combination of I₃⁻ ions derived from the iodine tincture and a reducing agent (here, ascorbic acid is used), reducing gold ions (Au⁺, Au³⁺) by an electrochemical substitution reaction (SLRR), and oxidizing platinum (Pt) to a platinum oxide (PtO).

Gold (Au) atoms deposited on the surface of crystal grains of platinum (Pt) do not migrate due to metal-metal bonds, as can be seen from the heteroepitaxial growth. On the other hand, gold (Au) reduced on the gold (Au) surface self-diffuses due to Rayleigh instability, and tends to form a spherical shape with a large radius of curvature and stable energy. Gold (Au) atoms in the second and subsequent layers migrate on the deposited surface to form an energetically stable crystalline state. Thereby, a single crystal region of gold (Au) having a nanoscale island structure is formed on the surface of a crystal grain of platinum (Pt).

Gold (Au) atoms adhering to the surface of a crystal grain of palladium (Pd) inter-diffuse into the palladium (Pd) and palladium (Pd) into the electroless plated gold (Au) while heteroepitaxially growing. Also, gold (Au) reduced on the gold (Au) surface self-diffuses due to Rayleigh instability, and tends to form a spherical shape with a large radius of curvature and stable energy. Gold (Au) atoms in the second and subsequent layers migrate on the deposited surface to form an energetically stable crystalline state. As a result, gold diffuses into crystal grains of palladium (Pd), and a crystal region consisting of an alloy in which Pd is diffused is formed in gold (Au) having a nanoscale island structure.

### 1-6-3. Pretreatment of Electroless Plating

The surface of the oxidized first metal member 110 may be subjected to a pretreatment for reduction before electroless plating. A pretreatment liquid containing an oxidizing agent and a reducing agent is used as the pretreatment. For example, iodine ions (I⁻, I₃⁻) derived from iodine tincture are used as the oxidizing agent, and a combination of L(+)-ascorbic acid (C₆H₈O₆) is used as the reducing agent. The pretreatment is performed by immersing first metal member 110 in such a pretreatment liquid. This pretreatment reduces the platinum oxide (PtO) formed on the surface of the first metal member 110 to form the surface of platinum (Pt), and increases the nucleation density in the electroless plating treatment.

### 2. Base Sequence Analyzer

In this section, an example of a device using a nanopore structure is shown. Specifically, an example of a device for analyzing the base sequence of DNA or RNA is shown. It is possible to use the nanopore structure 100 as an electrode in such an analyzer because it has conductivity.

### 2-1. Fifth Embodiment

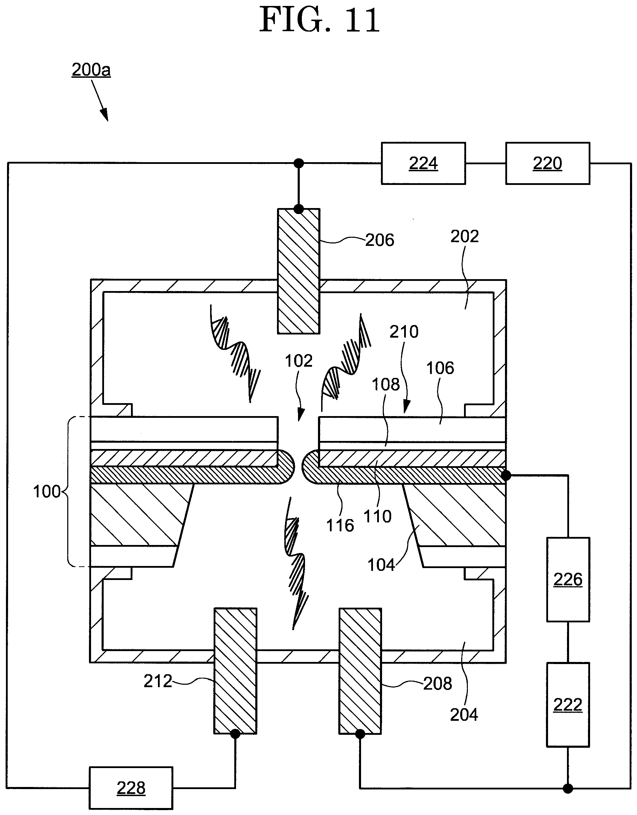

FIG. 11 schematically shows a cross-sectional structure of the base sequence analyzer 200a. The base sequence analyzer 200a has a cis chamber 202 in which a solution to be a sample is put and a trans chamber 204. The cis chamber 202 and the trans chamber 204 are filled with a liquid containing a sample (DNA, RNA, etc.) to be analyzed. Although not shown in FIG. 11, the cis chamber 202 may be provided with an inlet tube for supplying the sample solution, and the trans chamber 204 may be provided with an outlet tube for flowing the sample solution. Further, a flow path may be provided between the cis chamber 202 and the trans chamber 204 so that the sample solution is circulated across the nanopore structure 100.

A nanopore structure 100 is arranged between the cis chamber 202 and the trans chamber 204 to separate them. The nanopore structure 100 is arranged such that the surface on which the first metal member 110 and the second metal member 116 are formed faces the side of the cis chamber 202. The cis chamber 202 and the trans chamber 204 are connected by a nanopore 102. Note that the nanopore structure 100 can be applied to any of the first to fourth embodiments shown in this embodiment.

A liquid containing ions is used as the sample solution. The sample (DNA, RNA, etc.) is contained in a liquid containing ions. The solution is preferably an aqueous solution in which an electrolyte having a high ionization degree is dissolved, and a salt solution, for example, a potassium chloride aqueous solution, is used. For example, 1MKCl (or uniform salts such as NaCI, LiCI, etc.) and a pH buffer system (for example, so that the proteins used, such as protein nanopores, nucleases, etc., are not denatured) can be used according to the present invention (for controlling moving speed by varying the potential across the nanopore). In some embodiments, a pH buffer system is used to keep the pH in the range of 6.8 to 8.8 to maintain a substantially constant pH.

The surface of the nanopore structure 100 (the surface of the first metal member 110 and the surface of the second metal member 116) may be surface treated to enhance the wettability of the sample solution. The surface treatment can be applied with an oxygen plasma treatment or UV ozone treatment. DNA and RNA are negatively charged as a whole because OH groups bound to phosphate are ionized.

The cis chamber 202 is provided with a first electrode (working electrode) 206. The trans chamber 204 is provided with a second electrode (counter electrode) 208 and a fourth electrode (reference electrode 2) 212. The nanopore structure 100 is used as a third electrode (reference electrode 1) 210.

A first bias circuit 220 and a first current measurement circuit 224 are connected between the first electrode (working electrode) 206 and the second electrode (counter electrode) 208. A second bias circuit 222 and a second current measurement circuit 226 are connected between the third electrode (reference electrode 1) 210 and the second electrode (counter electrode) 208. A voltage measurement circuit 228 is connected between the first electrode (working electrode) 206 and the fourth electrode (reference electrode 2: e.g., Ag/AgCI reference electrode) 212 with a very large impedance and no current flows between the fourth electrode 212 and the first electrode 206. The base sequence analyzer 200a has a function of decoding the base sequence in a process of moving a sample (DNA, RNA, etc.) in a solution from the cis chamber 202 to the trans chamber 204 through the nanopore 102 by controlling the first electrode (working electrode) 206, the second electrode (counter electrode) 208, and the third electrode (reference electrode 1) to a predetermined potential.

The voltage difference measured by the voltage measuring circuit 228 between the cis chamber 202 and trans chamber 204 may be in the range of 70 mV to 200 mV in some embodiments. The voltage difference between the cis chamber 202 and the trans chamber 204 may be in the range of 80 mV to 150 mV in other embodiments. The appropriate voltage for the operation can be selected using conventional measurement techniques. The current (or voltage) due to the nanopore 102 can be easily measured using commercially available instruments.

The moving speed of single-stranded DNA (polynucleotides) depends on the voltage difference (or electric field strength) between the cis chamber 202 and trans chamber 204, and the reaction mixture in the cis chamber 202 into which the polynucleotides are introduced (e.g., placed on a solid phase membrane comprising one wall of the cis chamber 202) or the pH by the pH buffer system. The rate of capture of the polynucleotide by the nanopore 102 depends on the concentration of the polynucleotide. The voltage difference between the cis chamber and the trans chamber can be selected in some embodiments so that the conventional reaction mixture conditions for sequencing and the moving speed of the polynucleotide are within a desired range. The range of moving speeds, in some embodiments, includes speeds of less than 1000 nucleotides per second. The range of moving speed in other embodiments is 10 to 800 nucleotides per second, in other embodiments the range of moving speed is 10 to 600 nucleotides per second, in other embodiments the range of moving speed is 200 to 800 nucleotides per second, and in other embodiments the range of moving speed is 200 to 500 nucleotides per second. Similarly, other factors that affect moving speed, such as temperature, viscosity, ion concentration, etc., may be selected to obtain a moving speed within the ranges cited above.

### 2-1-1. Measurement of Ion Current

The potential of the first electrode (working electrode) 206 is V1, the potential of the second electrode (counter electrode) 208 is V2, and the potential of the third electrode (reference electrode 1) 210 is V3. First, the voltage V1 is applied to the first electrode (working electrode) 206 and the voltage V2 is applied to the second electrode (counter electrode) 208 by the first bias circuit 220 (V1 < V2), and the voltage V2-V1 is set, for example, in the range of 70 mV to 200 mV, more preferably in the range of 80 mV to 150 mV, and the potential difference between the first electrode (working electrode) 206 and the fourth electrode (reference electrode 2) 212 measured by the voltage measuring circuit 228 is controlled so that the ion current can be stably measured. When measuring only the ion current, the voltage measuring circuit between the fourth electrode and the first electrode may be opened. Further, when the sample (DNA, RNA, etc.) has a negative charge, the potential is controlled so that the voltage V3 of the third electrode (reference electrode 1) 210 becomes V1 ≦ V3 and V3 < V2.

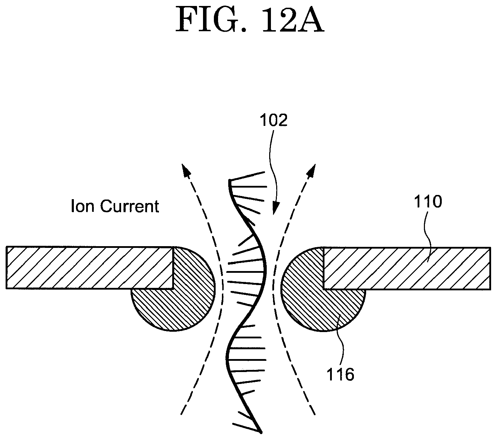

The sample (DNA, RNA, etc.) in the sample solution put in the cis chamber 202 is moved and collected to the vicinity of the nanopore 102 of the third electrode (reference electrode 1) 210 by applying the bias described above. The sample (DNA, RNA, etc.) is attracted to the side of the second electrode (counter electrode) 208 of the trans chamber 204 by passing through the through holes 113 while reducing the entanglement of the single strands. When the current between the first electrode (working electrode) 206 and the second electrode (counter electrode) 208 is measured by an ammeter, if the sample (DNA, RNA, etc.) is not present in the nanopore 102, only the ion current due to the solution used as the sample solution is measured. As shown in FIG. 12A, when a sample (DNA, RNA, etc.) is present in the nanopore 102, the cross-sectional area limits the ions that can pass through the nanopore 102, so that the ion current flowing through the nanopore 102 is reduced. This amount of change can be measured as a time series blocking current by a sample (DNA, RNA, etc.), and analysis such as detection of a base can be performed.

The step of collecting the sample (DNA, RNA, etc.) near the nanopore 102 can be separated from the step of passing the sample through the nanopore 102. In the step of collecting the sample (DNA, RNA, etc.) in the vicinity of the nanopore 102, the potential of the first electrode (working electrode) 206 and the third electrode (reference electrode 1) 210 is controlled so that V1 < V3. At this time, the potential V2 of the second electrode (counter electrode) 208 may be any potential, for example, V3 ≥ V2. The sample (DNA, RNA, etc.) in the sample solution is collected to the third electrode (reference electrode 1) 210 (i.e., in the vicinity of the nanopore 102) by applying such a bias.

Next, in the step of passing the sample (DNA, RNA, etc.) through the nanopore 102, the potential V3 of the third electrode (reference electrode 1) 210 and the potential V2 of the second electrode (counter electrode) 208 are controlled so that V3 < V2. At this time, the potential V1 of the first electrode (working electrode) 206 may be set to the same potential as V3. The large number of samples (DNA, RNA, etc.) can be passed in a short time by collecting the samples (DNA, RNA, etc.) in the vicinity of the nanopore 102 and passing the nanopore 102 thereafter. In this method, it is possible to control the timing of the start of detection of the blocking current, so that the base can be detected only during the time when the sample is passing through the nanopore 102, and efficient data collection is possible.

In order to allow the sample (DNA, RNA, etc.) to reach the nanopore 102 as quickly as possible, the potential difference between the potential V1 of the first electrode (working electrode) 206 and the potential V3 of the third electrode (reference electrode 1) 210 is preferably larger. On the other hand, in the step of measuring the ion current, the potential difference between the potential V3 of the third electrode (the reference electrode 1) and the potential V2 of the second electrode (the counter electrode) 208 affects the speed at which the sample (DNA, RNA, etc.) passes through the nanopore 102 and outputs an electric signal, and therefore, it is preferable to set an appropriate value according to the sampling speed of the first current measurement circuit 224. When the sample (DNA, RNA, etc.) has a positive charge, the relationship between the magnitude of the potential and that when the sample has a negative charge is reversed. For example, each potential is V1 ≥ V3 > V2.

### 2-1-2. Measurement of Tunnel Current

When the DNA or RNA as the sample has conductivity, a tunnel current can be measured at the third electrode (reference electrode 1) 210 provided with the nanopore 102 by using the characteristics of DNA or RNA.

The potential V1 of the first electrode (working electrode) 206 and the potential V2 of the second electrode (counter electrode) 208 are controlled so that the potential difference between the first electrode (working electrode) 206 and the fourth electrode (reference electrode 2) 212 measured by the voltage measuring circuit 228 is controlled to be V1 < V2, and a chemical potential is applied to the sample solution so that a tunnel current flows at the third electrode (reference electrode 1) 210, so that the tunnel current flows from the sample (DNA, RNA, etc.) to the third electrode (reference electrode 1).

When a tunnel current easily flows to the third electrode when a sample (DNA, RNA, etc.) is present (passing through) in the nanopore 102 compared to when the sample (DNA, RNA, etc.) is not present in the nanopore 102, the tunnel current corresponding to the base of the sample (DNA, RNA, etc.) can be measured by the second current measurement circuit 226. In this case, the third electrode (reference electrode 1) 210 is controlled to have a potential for controlling a chemical potential in which a tunnel current flows between the third electrode (reference electrode 2) and the fourth electrode (reference electrode 2) 212.

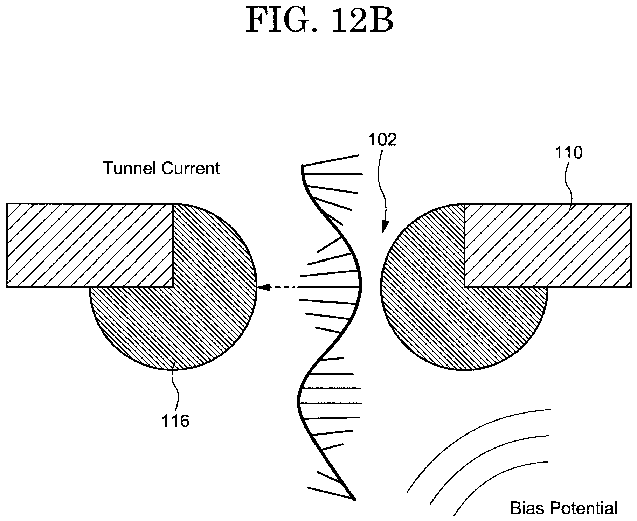

The tunnel current decays exponentially with the tunnel distance, making it very sensitive to the base structure, single-chain shape, and orientation through the nanopore. As schematically shown in FIG. 12B, when the radius of curvature of the second metal member 116 forming the nanopore 102 is made small, the tunnel current flowing from the base closest to the second metal member 116 becomes dominant when the sample (DNA, RNA, etc.) passes through the nanopore 102 due to this characteristic, and the base sequence can be accurately read out by analyzing the tunnel current in a time series.

### 2-2. Sixth Embodiment

DNA contains four bases, adenine (A), thymine (T), guanine (G), and cytosine (C), and the double helix is formed by base pairs formed by A-T (two hydrogen bonds) and G-C (three hydrogen bonds). The pentose of RNA (ribonucleic acid), another type of nucleic acid, is ribose, and the base contains uracil (U) instead of T. The sequence of the bases can be read by emission spectrometry or absorption spectrometry by chemisorbing a molecule containing a group whose emission characteristic or absorption characteristic changes when combined with these base components onto the surface of the second metal member 116 forming the nanopore 102.

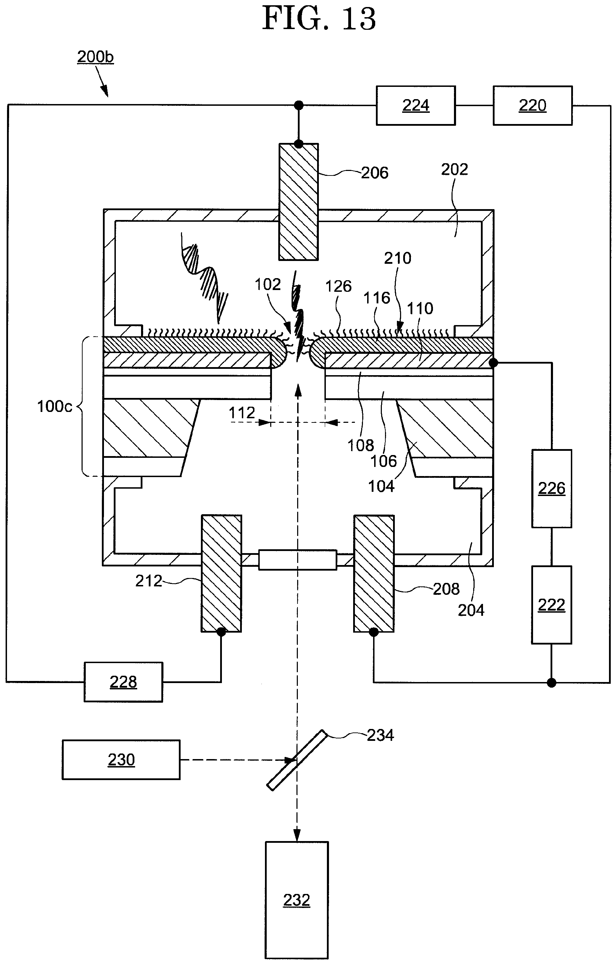

FIG. 13 shows an example of a base sequence analyzer 200b which can be used for emission analysis. The base sequence analyzer 200b is equipped with a nanopore structure 100c shown in the third embodiment between the cis chamber 202 and the trans chamber 204. An excitation light source 230 and a detector 232 are provided on the side of the trans chamber 204, and a beam splitter 234 is configured to irradiate a portion of the nanopore 102 with light and to observe light emission by the excitation light. A spectroscope is used as the detector 232, and the light emitted by the excitation light is spectrally detected. Although not shown in FIG. 13, an optical member such as a condenser lens may be provided on the optical path.

A group emitting light when the excitation light is irradiated from the back surface side (the trans chamber 204 side) of the nanopore 102 chemically adsorbs only on the surfaces of the second metal members 116a, 116b, so that a change in light emission can be observed only in the nanopore 102. Thus, when a molecule containing a group whose luminescent property or absorption property changes for each base is chemically adsorbed on a part of the nanopore 102, and excitation light is irradiated from the rear surface to observe the luminescent property or absorption property, bases can be discriminated one by one when a single strand of DNA passes through the nanopore 102, and the base sequence of DNA can be read out. In this case, the self-assembled monolayer 126 is arranged only on the side of the cis chamber 202, so that the sequence of bases can be accurately decoded.

The self-assembled monolayer 126 may be intercalated with phosphor molecules that label specific bases contained in DNA and RNA. For example, the self-assembled monolayer 126 may be intercalated with different phosphor molecules so that different phosphors correspond to each of the four bases (adenine, guanine, thymine, cytosine) contained in the DNA. The self-assembled monolayer 126 having such an intercalated phosphor molecule can perform fluorescence analysis when the base sequence of a sample (DNA, RNA, etc.) is decoded as a time-series optical signal by using the nanopore 102.

It is preferable that the self-assembled monolayer 126 is formed only on the narrowing lower surface portion of the nanopore and the surface of the narrowing portion on the side of the through hole 113 of the insulating layer 106, when forming the self-assembled monolayer 126 containing phosphor molecules. Thus, the self-assembled monolayer 126 containing phosphor molecules is formed only in the nanopore portion on the cis chamber side.

The fluorescent material may be, for example, a fluorescein dye, a rhodamine dye, a xanthene dye, a cyanine dye, or the like. To determine the monomer sequence of DNA, the monomer is labeled with a fluorescent label.

It is preferable that the excitation light is irradiated from the side of the trans chamber 204, but the excitation light may be irradiated from the side of the cis chamber 202. When a self-assembled monolayer is formed so that a fluorescent dye is disposed only in the nanopore 102, fluorescence that changes in combination with a base of DNA can be detected with a good SN ratio. When a self-assembled monolayer is formed so that a fluorescent dye is contained only in the nanopore 102, the fluorescence that changes in combination with a base of DNA can be detected with a high SN ratio. The fluorescence is preferably detected from the incident side by using an optical system for irradiating excitation light and a half mirror. The nanopore 102 includes gold (Au), and the gold (Au) surface amplifies the excitation light intensity by surface plasmon enhancement. Thus, the photons of the excitation light can efficiently excite the fluorescent dye. When excitation light is detected, if a metal is present in the vicinity of the dye, it may be quenched. This quenching can be prevented by appropriately controlling the distance between the dye and the nanopore by the self-assembled monolayer 126.

The self-assembled monolayer 126 is 0.3 nm to 5 nm in length, so the effective pore size of the nanopore 102 is reduced by that length. It is preferable that the affinity between the self-assembled monolayer 126 and the DNA base prevents the quenching between the second metal member 116 and the fluorescent dye in comparison with the case where the surface of the nanopore 102 is only a metal.

A method for analyzing a base sequence according to an embodiment of the present invention can be carried out by: (a) a step of restricting the monomer so that different types of monomers of the polymer are labeled with different optical labels that generate differentiable optical signals and the through holes 113 and nanopores 102 of the insulating layer 106 in connection with the nanopores 102 move in a row; (b) a step of detecting a time series change in the optical signal from the monomer as the polymer passes through the nanopore 102; (c) a step of separating optical signals from different types of monomers; and (d) a step of determining the sequence of the monomers from a time series change in the optical signal.

A fluorescent signal is used here as an optical signal. It is considered that the fluorescence signal generated in the transition period is due to the presence of one or more freely rotatable dipoles of the fluorescent label that emerged from the nanopore 102, allowing the fluorescent label to generate a fluorescence signal, for example, after direct excitation or by excitation via fluorescence resonance energy transfer (FRET). The polynucleotide, in some embodiments, is a single-stranded polynucleotide, such as DNA or RNA, but is single-stranded DNA. Some embodiments include a method for determining the nucleotide sequence of the polynucleotide by recording the signal generated by the fluorescent label as the polynucleotide moves through the nanopore 102, one at a time, as it exits the nanopore.

The second metal member 116 is made of gold (Au) or the like, so that the excitation frequency can be increased by surface plasmon resonance. In particular, the excitation frequency of the nanopore 102 is extremely high because the second metal member 116 surrounds the single-stranded DNA. Moreover, the radius of curvature of the second metal member 116 forming the nanopore 102 is 1 nm to 20 nm, so that the spatial resolution for increasing the excitation frequency can be reduced, and the base sequence of the single-stranded DNA passing through the nanopore 102 can be easily separated from the time-series optical signal due to the high excitation frequency at the nanopore 102 when the DNA passes through the nanopore 102.

The through hole 112 of the insulating layer 106 is connected to the nanopore 102. The hole diameter of the through hole 112 becomes equal to the hole diameter of the first metal member 110 and the base metal layer 108 by using reactive ion etching (RIE) capable of anisotropic etching. Therefore, the width of the through hole 112 is 50 nm or less, preferably 20 nm or less, and more preferably 10 nm or less. The through hole 112 in the insulating layer 106 is arranged as a cis chamber side in which DNA exists, and when the DNA is passed through the nanopore 102 and transported to the trans chamber on the opposite side, the width of the through hole 112 of the insulating layer 106 is narrowed, entangled DNA is untangled, and the entangled DNA is led to the nanopore 102 as single-stranded DNA, which is effective as a micro channel. When the cis chamber is used as the upper portion, it is preferable that the structure shown in FIG. 1A and FIG. 1B or FIG. 3A and FIG. 3B is upside down so that the DNA passes through the upper cis chamber from the upper cis chamber to the trans chamber with the through hole 112 of the insulating layer 106 on the upper side and the nanopore 102 on the lower side.

### 2-3. Seventh Embodiment

The base sequence analyzer shown in FIG. 13 may use a laser light source as the excitation light source 230 and a spectroscopic detector for detecting Raman scattered light as the detector 232. In this case, the self-assembled monolayer 126 provided on the nanopore structure 100c is loaded with a material whose Raman shift is changed by interaction with a base.

A method for analyzing a base sequence according to an embodiment of the present invention can be carried out by: (a) a step of restricting the monomer so that the through holes 113 and the nanopore 102 of the insulating layer 106 connected to the nanopore move in a row, by labeling the monomer of a different kind of polymer by generating a distinguishable Raman spectrum by surface plasmon enhancement of the nanopore portion by the second electrode material; (b) a step of detecting a time-series change in the Raman spectrum as an optical signal from the monomer as the polymer passes through the nanopore; (c) a step of separating Raman spectra from different kinds of monomers; and (d) a step of determining the sequence of the monomers from a time series change in the Raman spectrum.

The intensity of the surface plasmon resonance Raman scattering light can be greatly increased when the single-stranded DNA passes through the nanopore portion, since the constricted portion of the nanopore 102 is composed of a metal such as gold (Au). The specific bases in DNA and RNA have Raman spectra due to their structural differences. Especially, the effect of surface plasmon enhancement is extremely high, since the second metal member 116 surrounds the single-stranded DNA in the portion of the nanopore 102. Further, it is possible to reduce the spatial resolution for increasing the excitation frequency since the radius of curvature of the nanopore 102 is 1 nm to 20 nm, to facilitate the spectroscopic evaluation of a time-series optical signal by increasing the intensity of Raman scattered light at a portion of the nanopore 102 when the DNA passes through the nanopore 102, and to evaluate the base sequence of single-stranded DNA passing through the nanopore 102 with high resolution.

### 2-4. Eighth Embodiment

As the nanopore structure for dividing the cis chamber 202 and the trans chamber 204, a nanopore structure 100d in which a plurality of electrodes provided with nanopores are arranged can be used as shown in the fourth embodiment. The following description will focus on the differences from the base sequence analyzer 200a shown in FIG. 11.

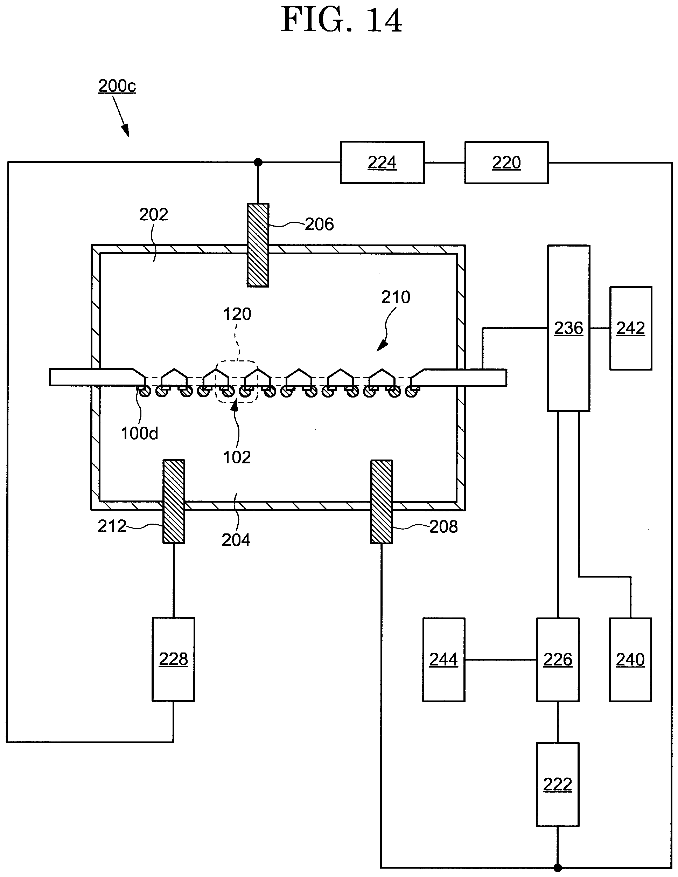

A base sequence analyzer 200c shown in FIG. 14 is equipped with a nanopore structure 100d having a plurality of electrodes 120 with nanopores between a cis chamber 202 and a trans chamber 204. The plurality of electrodes 120 with nanopores are electrically isolated from each other, and are connected to a selection circuit 236 by individual wiring (not shown). The selection circuit 236 is connected to the second bias circuit 222 and a third bias circuit 240. The second current measurement circuit 226 for measuring the current using the third electrode (reference electrode 1) 210 may be arranged between the selection circuit 236 and the second bias circuit 222. The selection circuit 236 is connected to the second bias circuit 222 and the third bias circuit 240. The second current measurement circuit 226 for measuring the current using the third electrode (reference electrode 1) 210 may be arranged between the selection circuit 236 and the second bias circuit 222.

The third bias circuit 240 applies a bias voltage of a different level to the second bias circuit 222. The selection circuit 236 has a function of switching the connection between each of the plurality of electrodes 120 with nanopores and the second bias circuit 222 and the third bias circuit 240. The selection circuit 236 includes, for example, a switch circuit composed of an analog switch or the like. A control circuit 242 for controlling the operation of the selection circuit 236 and a storage circuit 244 for storing data measured by the second current measurement circuit 226 may be included.

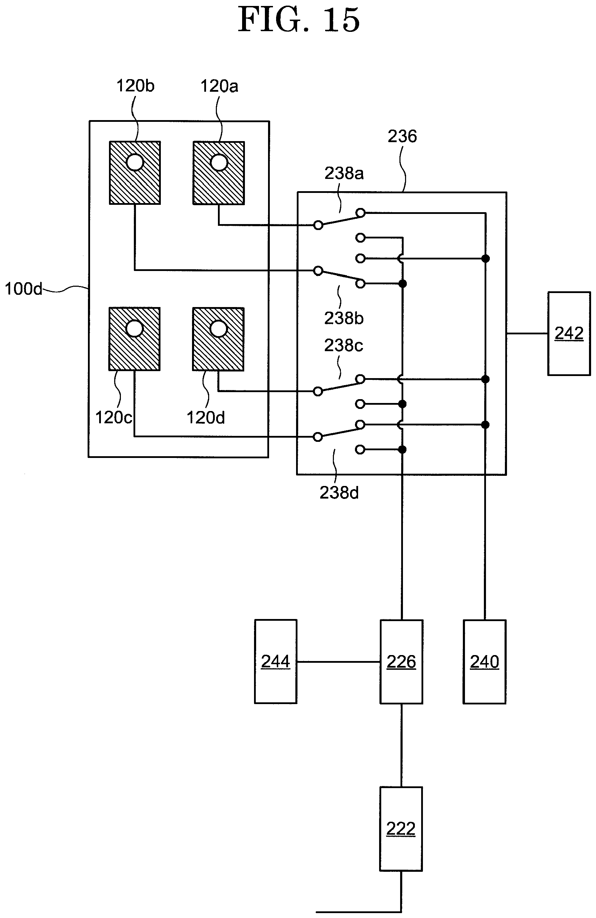

FIG. 15 shows an example of a connection between the plurality of electrodes 120 with nanopores and the selection circuit 236. FIG. 15 schematically illustrates the nanopore structure 100d is arranged with four nanopore electrodes 120a to 120d. The selection circuit 236 includes switches 238a to 238d. The operation of the switches 238a to 238d is controlled by the control circuit 242. The nanopore electrode 120a is connected to the switch 238a. The switch 238a has a function of switching the connection between the second bias circuit 222 and the third bias circuit 240. The nanopore electrodes 120b to 120d are also connected to switches 238b to 238d.

It is assumed that the second bias circuit 222 has a function of applying a potential V3 to the nanopore electrodes 120a to 120d, and the third bias circuit 240 has a function of applying a potential V4. As explained in section 2-2, when the potential V1 of the first electrode (working electrode) 206, the potential V2 of the second electrode (counter electrode) 208, and the potential V3 of the third electrode (reference electrode 1) 210 are controlled so that the potential V1 < V3 and the potential V3 < V2, the potential V4 is set to be the same as or lower than the potential V1. It can also be referred to as a suppression potential (or suppression voltage), since the potential V4 is a potential for preventing an ion current from flowing.

As shown in FIG. 15, when the nanopore electrode 120a is connected to the second bias circuit 222 and the other nanopore electrodes 120b to 120d are connected to the third bias circuit 240 in the base sequence analyzer 200c shown in FIG. 14, the ion current flows to the nanopore electrode 120a, and the ion current does not flow or the ion current flows at an almost negligible level to the other nanopore electrodes 120b to 120d.

### 2-4-1. Measurement of Ion Current

The connection terminals of the nanopore electrodes 120a to 120d are sequentially switched by the selection circuit 236, and the ion current is measured by the second current measurement circuit 226 according to the timing, so that it is possible to know to which nanopore in the nanopore structure 100d the sample (DNA, RNA, etc.) has passed (or has begun to pass).

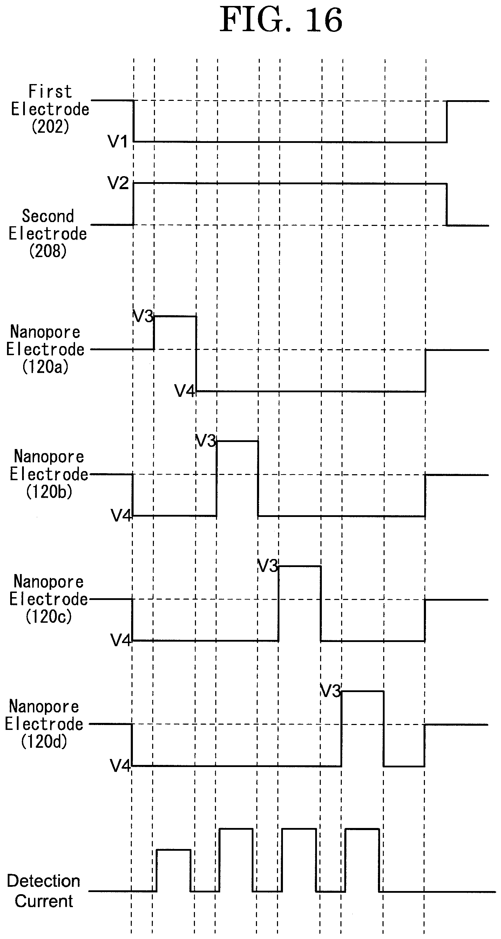

FIG. 16 shows an example of a timing chart of such an operation. The first electrode (working electrode) 206 is set to a potential V1, the second electrode (counter electrode) 208 is set to a potential V2, the nanopore electrode 120a is set to a potential V3, and the other nanopore electrodes 120b to 120d are set to a potential V4. The first electrode (working electrode) 206 is set to a potential V1, the second electrode (counter electrode) 208 is set to a potential V2, the nanopore electrode 120a is set to a potential V3, and the other nanopore electrodes 120b to 120d are set to a potential V4. After a certain period of time, at the next timing, the nanopore electrode 120a is set to the potential V4 and the nanopore electrode 120b is controlled to the potential V3. Hereinafter, it is possible to sequentially measure the ion current flowing through the nanopore electrodes 120a to 120d by sequentially repeating the control of the potential. As shown in FIG. 16, the ion current is sequentially (serially) measured from the second current measurement circuit 226. It is possible to evaluate whether or not a blocking current is flowing from the magnitude of the ion current. The data measured by the second current measurement circuit 226 can also be stored in the storage circuit 244.

### 2-4-2. Evaluation of Nanopore Array

The ease of ion current flow in the device shown in FIG. 14 depends on the pore size of the 102 nanopores. It is possible to evaluate the nanopore 102 by utilizing the characteristics thereof. The pore diameter of the nanopore 102 is preferably uniform in the nanopore structure 100d, but variations in the pore diameter may be included due to manufacturing variations. In such a case, the quality of the nanopores 102 can be evaluated or the nanopores 102 can be classified by the methods described below.

The cis chamber 202 and the trans chamber 204 are filled only with a solution containing no sample (DNA, RNA, etc.). In this condition, the ion current is sequentially measured for each electrode with nanopores as described with reference to FIG. 16. The ion current is measured by the second current measurement circuit 226, and the measured value may be stored in the storage circuit 244 corresponding to the addresses of the electrodes 120a to 120d with nanopores. Referring to a standard nanopore (as a predetermined reference value), a larger ion current than the reference value indicates that the pore diameter of the nanopore 102 is larger than the reference value, and a smaller ion current indicates that the pore diameter is smaller than the reference value.

According to the above evaluation, the nanopores 102 classified in one nanopore structure 100d can be selectively used for measuring a sample or not according to the criterion. For example, the nanopore determined to be defective may be left at the suppression potential V4 so as not to be used for measuring the sample.

As described above, according to the present embodiment, the base sequence of the sample can be efficiently decoded by arranging a plurality of electrodes 120 with nanopores in the nanopore structure 100d that partitions the cis chamber 202 and the trans chamber 204. In this case, it is possible to analyze the sample in the nanopore of interest while sharing the cis chamber 202 and the trans chamber 204 by individually controlling the potential of each electrode 120 with the nanopore.

### 2-5. Ninth Embodiment

According to the configuration shown in the fifth to seventh embodiments, it is possible to apply a pulse voltage to the third electrode (reference electrode 1) 210 by using the second bias circuit 222 as a pulse power source so that the sample passing through the nanopore at the time of applying the pulse voltage can pass through each base. Thereby, when measuring the ion current, the tunnel current, the luminescence/absorption analysis, and the Raman shift, the evaluation can be performed one base at a time. This method can reduce reading errors and accurately decode the base sequence.

Since the ion current, tunnel current, luminescence/absorption spectrometry, and Raman shift can be measured independently, the accuracy of the base sequence can be improved by measuring a plurality of combinations of these measurements.

Since voltage measurement, voltage application, and current measurement between various electrodes can be operated by a battery, the nanopore electrode and the battery can be combined to form a portable DNA and RNA analyzer.

### EXAMPLE

### EXAMPLE 1

This example shows the fabrication of nanopore structures. The nanopore structures were fabricated according to the process described in Section 1-5. The outline of the fabrication conditions was as follows.
(1) A silicon wafer (300 µm in thickness) on which a 100 nm oxide film (SiO₂) is formed on both surfaces is used, and a positive photoresist (OFPR-800) is coated on the back surface thereof.
(2) A resist pattern for forming a cavity of 100 × 100 µm² on the back surface of a silicon wafer is formed by a photolithography process.
(3) The backside oxide is etched using an ICP-RIE (Inductive Coupled Plasma Reactive Ion Etching) process and then the photoresist is removed. The patterned backside oxide layer is used as a hard mask for the next etching process.
(4) The silicon wafer (300 µm) is removed by the ICP-RIE process to form a cavity on the backside.
(5) A positive EBL resist (ZEP-520A) is coated on the surface of a silicon wafer.
(6) Resist patterning forms to fabricate the nanopore structure and the electrode lead. An electron beam is not exposed to the nanopore part, and the resist is exposed by irradiating the periphery of the region where the nanopore is formed with the electron beam. An electron beam is irradiated so that the electrode lead and the peripheral part of the nanopore are connected. The region to be the nanopore, to which no electron beam is irradiated, may be square or circular.
(7) Metal layers is deposited in the order of titanium (Ti) and platinum (Pt) by electron beam evaporation.
(8) The resist is removed by a lift-off process to expose the surface of the oxide layer (SiO₂) at the portion where the nanopore is to be produced.
(9) A pattern of an electrode pad (100 × 100 µm²) for independently applying a voltage to the nanopore is patterned with a photoresist.
(10) Titanium (Ti) and platinum (Pt) layers for electrode pads are formed by electron beam evaporation.
(11) A positive EBL resist is coated on the surface of a silicon wafer, and a portion where a nanopore exists is patterned.
(12) The exposed portion of the oxide layer (SiO₂) on the surface is processed by the ICP-RIE process using a titanium (Ti)/platinum (Pt) layer mask to form penetrated nanopores.
(13) Gold plating is performed on a titanium (Ti)/platinum (Pt) electrode having a hole on the surface of the nanopore by an electroless plating process, and the hole size is reduced to 2 nm or less.
(14)The EBL resist is removed.

In the above-described fabrication process, samples having different electron beam drawing areas in (6) were evaluated. Specifically, a pattern in which an area for exposing an oxide film (SiO₂) without irradiating an electron beam was changed was produced on the same substrate, and the pore diameter of the nanopore was evaluated by a scanning electron microscope (SEM).

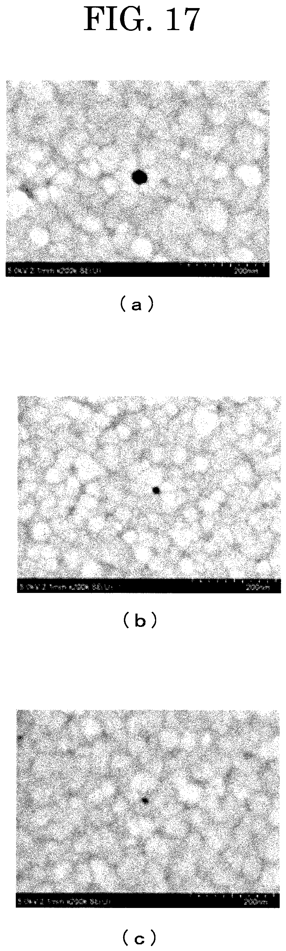

FIG. 17 shows (a), (b), and (c) are SEM images of samples with different areas of oxide layer (SiO₂) exposed by not applying the electron beam. The sample (a) has a square shape and an area not irradiated with an electron beam is 100 × 100 nm², the sample (b) is 60 × 60 nm², and the sample (c) is 40 × 40 nm². The size of the nanopore in each sample is 40 nm for sample (a), 17 nm for sample (b), and 10 nm for sample (c). As a result, it is shown that the size of the nanopore after electroless plating decreases as the drawing pattern of the electron beam decreases. In this plating condition, since the nucleation density of gold (Au) is high, the grains are interconnected. The flat part is covered with gold. In addition, the exposed portion of the oxide layer (SiO₂) is a circular nanopore instead of a square. This suggests that nanopores tend to be circular because they are more energetically stable than squares. The cross-section perpendicular to the substrate surface is a circular structure in the nanopore area, similar to the inner hole portion of a doughnut. Since the through hole has a protruding shape when compared with the flat portion, plating easily advances in the circular cross section of the nanopore and a raised structure is formed compared with the flat portion. However, there are cases in which the structure is not higher than the flat portion. The circular structure of the nanopore has the same radius of curvature in the range of at least 180 degrees, and the circle penetrates the initial pore portion of the boundary between first metal member 110 and the base metal film 108.

### EXAMPLE 2

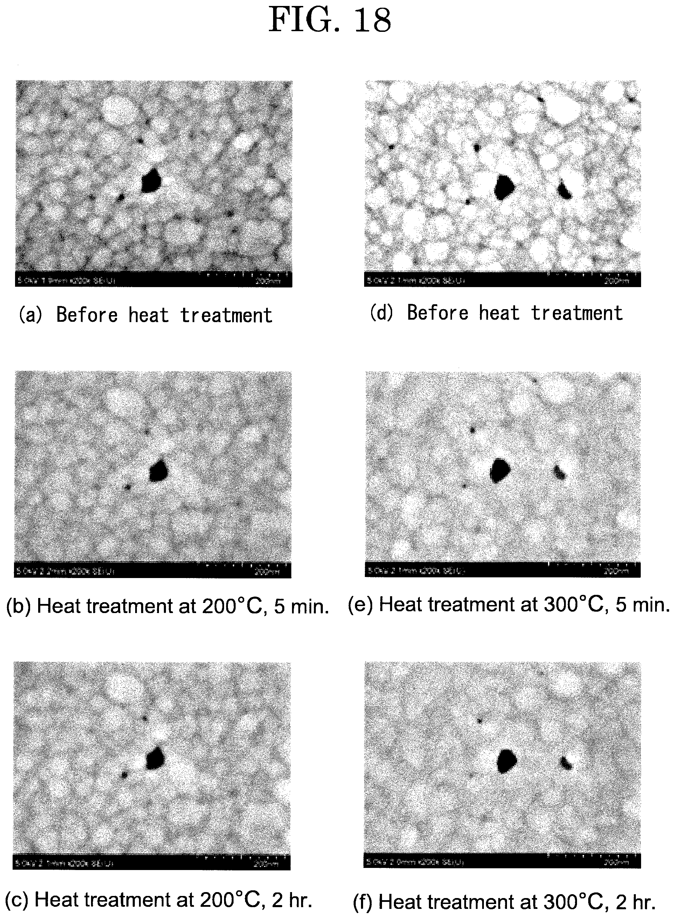

This embodiment shows the result of evaluating the heat resistance of the nanopore structure. FIG. 18 shows a result of a heat treatment of samples in which nanopores are formed at 200°C and 300°C as SEM images. FIG. 18 shows samples (a) and (d) before heat treatment, (b) after heat treatment at 200°C for 5 minutes, (c) after heat treatment at 200°C for 2 hours, (e) after heat treatment at 300°C for 5 minutes, and (f) after heat treatment at 300°C for 2 hours. The gold (Au) surface is smooth when the sample after the heat treatment is observed. On the other hand, it can be seen that there is no change in the shape of the central nanopore. The results showed that the nanopores were resistant to heat treatment at 300°C. It has also been found that there is no change in the shape even when an oxygen plasma treatment is applied. It is considered that this heat resistance of the nanopore formed of gold (Au) is caused by heteroepitaxial growth on platinum (Pt).

When unraveling the double helix structure of DNA into single chains, a heating technique at about 65°C is used. It is found that the nanopore fabricated in this embodiment can endure 300°C, and therefore the measurement cell containing the nanopore can be heated to form a single chain.

### INDUSTRIAL APPLICABILITY

The nanopore structure according to an embodiment of the present invention can be used in a biological sample analyzer. The biological sample analyzer of an embodiment of the present invention has a nanopore structure and can be used for sequencing (i.e., DNA sequencer) DNA, RNA, etc.

### Supplementary Note

A method of sequence analysis based in whole or in part on the exemplary embodiments of the invention disclosed herein is appended below.

A method for analyzing a base sequence according to an embodiment of the present invention includes the steps of: (a) a step of restricting the monomer so that different types of monomers of the polymer are labeled with different optical labels that generate differentiable optical signals and the through holes and nanopores of the insulating layer in connection with the nanopores move in a row; (b) a step of detecting a time series change in the optical signal from the monomer as the polymer passes through the nanopore; (c) a step of separating optical signals from different types of monomers; and (d) a step of determining the sequence of the monomers from a time series change in the optical signal.

A method for analyzing a base sequence according to an embodiment of the present invention includes the steps of: (a) a step of restricting the monomer so that the through holes 113 and the nanopore 102 of the insulating layer 106 connected to the nanopore move in a row, by labeling the monomer of a different kind of polymer by generating a distinguishable Raman spectrum by surface plasmon enhancement of the nanopore by the second electrode material; (b) a step of detecting a time-series change in the Raman spectrum as an optical signal from the monomer as the polymer passes through the nanopore; (c) a step of separating Raman spectra from different kinds of monomers; and (d) a step of determining the sequence of the monomers from a time series change in the Raman spectrum.

### REFERENCE SIGNS LIST

100: nanopore structure, 102: nanopore, 104: support member, 106: insulating layer, 108: base metal layer, 110: first metal member, 112: through hole, 113: through hole, 114: crystal grain, 115: amorphous region, 116: second metal member, 118: crystal region, 119: amorphous region 120: nanopore electrode, 122: wiring, 124: electrode pad, 126: self-assembled monolayer, 130: silicon substrate, 131, 132: insulating layer, 134: opening, 140: photoresist, 142: photoresist, 143: resist pattern, 144: photoresist, 145: resist pattern, 200: base sequence analyzer, 202: cis chamber, 204: trans chamber, 206: first electrode (working electrode), 208: second electrode (counter electrode), 210: third electrode (reference electrode 1), 212: fourth electrode (reference electrode 2), 220: first bias circuit, 222: second bias circuit, 224: first current measurement circuit, 226: second current measurement circuit, 228: voltage measurement circuit, 230: excitation light source, 232: detector, 234: beam splitter, 236: selection circuit, 238: switch, 240: third bias circuit, 242: control circuit, 244: storage circuit

## Claims

1. A nanopore structure, comprising:
a first metal member having a thin plate and a through hole; and
a second metal member formed to narrow a diameter of the through hole,
wherein the first metal member and the second metal member form a nanopore having a pore diameter of 10 nm or less.

2. The nanopore structure according to claim 1, wherein the pore diameter of the nanopore is 5 nm or less and 1 nm or more.

3. The nanopore structure according to claim 1, wherein the second metal member covers at least a portion of an upper surface of the first metal member and a side wall of the through hole.

4. The nanopore structure according to claim 1, wherein the second metal member has a curved surface having a spherical shape or a columnar shape at a portion overlapping an end portion of the through hole.

5. The nanopore structure according to claim 4, wherein a radius of curvature of the curved surface is equal to or less than a film thickness of the first metal member.

6. The nanopore structure according to claim 1, wherein the first metal member includes a polycrystalline structure, and the second metal member includes a crystal region heteroepitaxially grown from at least one crystal grain included in the first metal member.

7. The nanopore structure according to claim 1, wherein the second metal member includes a plurality of island structures surrounding the through hole.

8. The nanopore structure according to claim 1, wherein the second metal member is a membrane structure continuous on the first metal member, and the membrane structure surrounds the through hole,
wherein a thickness of a portion of the nanopore is thicker than the thickness of other portions of the membrane structure.

9. The nanopore structure according to claim 1, wherein the first metal member is one of platinum (Pt), palladium (Pd), rhodium (Rd), ruthenium (Ru), osmium (Os), and iridium (Ir), and the second metal member is gold (Au).

10. The nanopore structure according to claim 1, further comprising a selfassembled monolayer provided on a surface of the second metal member.

11. The nanopore structure according to claim 10, wherein the selfassembled monolayer includes an intercalating dye corresponding to each base of DNA or each base of RNA.

12. The nanopore structure according to claim 1, further comprising an insulating layer formed with the first metal member and the second metal member forming the nanopore,
wherein the insulating layer includes a second through hole overlapping the nanopore.

13. The nanopore structure according to claim 1, wherein the second through hole has a hole diameter of 20 nm or less.

14. A base sequence analyzer, comprising:
a cis chamber and a trans chamber;
a nanopore structure partitioning the cis chamber and the trans chamber;
a first electrode provided in the cis chamber; and
a second electrode and a third electrode provided in the trans chamber,
wherein the nanopore structure includes:
a first metal member having a thin plate structure and a through hole;
a second metal member arranged to narrow a diameter of the through hole, and
the first metal member and the second metal member form a nanopore having a pore diameter of 10 nm or less.

15. The base sequence analyzer according to claim 14, further comprising a micro-Raman spectroscopy system provided on a side of the trans chamber.

16. The base sequence analyzer according to claim 14, wherein the nanopore structure includes at least one fourth electrode formed of the first metal member and the second metal member.

17. The base sequence analyzer according to claim 16, wherein the nanopore structure is detachably.
